# EUROPÄISCHE PATENTSCHRIFT

(11) **EP 2 846 763 B1**
(45) Veröffentlichungstag und Bekanntmachung des Hinweises auf die Patenterteilung: **08.02.2017**
(21) Anmeldenummer: 13715896.0
(22) Anmeldetag: 05.04.2013
(51) Int. Cl.: A61K 8/34, A61K 8/35, A61K 8/37, A61K 8/39, A61K 31/045, A01N 35/06, A01N 37/02, A61Q 5/00, A61Q 11/00, A61Q 15/00, A61Q 17/00, A61Q 19/00, A61K 9/00, C07C 43/196, A61K 31/075, A61K 31/121, A61K 31/22, A01N 31/06, A01N 35/02, A01N 37/36

(54) **VERWENDUNG VON CYCLOHEXANOLETHERN MIT ANTIMIKROBIELLEN EIGENSCHAFTEN**
USE OF ANTIMICROBIAL ETHERS OF CYCLOHEXANOL
UTILISATION D'ETHERS DE CYCLOHEXANOL ANTIMICROBIENS

(30) Priorität: 08.05.2012 DE 102012008892; 16.08.2012 DE 102012016191
(43) Veröffentlichungstag der Anmeldung: 18.03.2015
(73) Patentinhaber: Merck Patent GmbH, 64293 Darmstadt (DE)
(72) Erfinder: RUDOLPH, Thomas, 64291 Darmstadt (DE); EISENBERG, Sylvia, 64646 Heppenheim (DE)
(86) Internationale Anmeldenummer: PCT/EP2013/001011
(87) Internationale Veröffentlichungsnummer: WO 2013/167220

(56) Entgegenhaltungen:
- EP-A1- 2 014 273
- EP-A2- 0 219 257
- WO-A1-2011/050871
- FR-A1- 2 187 227
- GB-A- 1 315 626
- JP-A- 2004 059 536
- JP-A- 2007 291 049
- JP-A- 2008 050 271
- US-A- 4 767 883
- US-A1- 2003 232 893

## Beschreibung

Die vorliegende Erfindung betrifft : a) die nicht-therapeutische Verwendung mindestens eines Cyclohexanoletherderivats der Formel I, wie in Anspruch 1 definiert, als antimikrobieller Wirkstoff oder als Deodorant- oder Antitranspirantwirkstoff; b) die Verbindungen der Formel I, wie in Anspruch 3 definiert, zur Verwendung als antimikrobieller Wirkstoff in pharmazeutischen Formulierungen, Medizinprodukten oder als Anti-Akne- und/oder Antischuppenwirkstoff; c) die Zubereitungen enthaltend diese Verbindungen und ein weiterer Konservierungsstoff und/oder antimikrobieller Wirkstoff und/oder ein Antioxidant; und d) die ausgewählte Cyclohexanoletherderivate, wie in Anspruch 16 definiert.

Mikrobielle Kontaminierung stellt ein wesentliches Problem in unserem täglichen Leben dar, zum Beispiel in Zusammenhang mit kosmetischen oder pharmazeutischen Produkten, Lebensmitteln, Oberflächen in Bädern oder Küchen oder Operationsbesteck. Gewöhnlich werden Konservierungsmittel verwendet, um mikrobielle Kontaminierung zu vermeiden.

Antimikrobielle Wirkstoffe sind jedoch nicht nur als Konservierungsmittel notwendig. Auch für viele kosmetische Anwendungen spielen antimikrobielle Wirkstoffe eine wichtige Rolle:
Schuppenbildung ist eine bei der Bevölkerung weit verbreitete Störung der Kopfhaut, die meist von leichtem bis mittlerem Juckreiz begleitet wird. Die Bildung von solchen gewöhnlichen Kopfschuppen ist nicht als Hautkrankheit im allgemeinen Sinne aufzufassen. Schuppen können durch Kopfhautstörungen entstehen, die beispielsweise durch übermäßige Sonnenaussetzung, Umwelteinflüsse aus der Luft oder kosmetische Haarprodukte ausgelöst werden können. Die Kopfschuppen entstehen in diesem Fall durch eine Überproduktion von Hornzellen, ausgelöst durch winzige Entzündungsherde der Kopfhaut, deren Ursache z.B. eine vermehrte Keimbesiedlung mit Pilzen (wie *Malassezia furfur* oder *Malassezia globosa)* oder Bakterien ist. Die unvollständig ausgereiften Hornzellen schilfern als Folge verfrüht in größeren Zellverbänden (Schuppen) ab. Da durch den Hautzellenverlust die äußerste Hautschicht dünner wird, führt Schuppenbildung zu einer erhöhten Empfindlichkeit der Kopfhaut, welche sich in Juckreiz und Rötungen äußert.

Unter Akne versteht man eine Hautstörung, die sich in entzündeten Papein, Pusteln oder Knoten äußert, hervorgerufen durch eine verstärkte Talkproduktion und Verhornungsstörung der Haut. Die Entzündungen können mit Rötung, Schwellung und Druckschmerzen verbunden sein. Neben genetischer Prädisposition, können Androgene, komedogene Substanzen (z.B. in Kosmetika), Rauchen, Stress oder eine übermäßige Besiedlung der Haut mit Bakterien mögliche Ursachen für Aknebildung sein. Akne kann beispielsweise durch Mikroorganismen wie *Propionibacterium acnes, Propionibacterium granulosum* oder *Staphylococcus epidermidis* ausgelöst werden. *Propionibacterium acnes* ist ein Bakterium, welches gewöhnlicherweise die Haut bevölkert und sich von Sebum ernährt. Akne kann beispielsweise entstehen, wenn die Zahl dieser Bakterien erhöht ist. Die Gegenwart von Bakterien in den Follikeln führt zu Entzündungsreaktionen, welche sich in Form von roten Knoten oder Pusteln äußert. Die Produktion von freien Fettsäuren durch die Bakterien fördert weiterhin die Entzündungsreaktion im Follikel.

Achselschweiß enthält neben Wasser und Salz viele weitere Substanzen (wie Fette, Aminosäuren, Zucker, Milchsäure, Harnstoff etc.). Frisch gebildeter Schweiß ist geruchlos; der typische Schweißgeruch entsteht erst durch die Einwirkung von Hautbakterien auf den Schweiß, welche diesen zersetzen. Beispiele für solche Bakterien sind *Staphylococcus, Corynebacterium* oder *Malassezia.* In Deodorants werden deshalb neben Geruchsstoffen und Antitranspirantien gewöhnlich auch antimikrobielle Stoffe eingesetzt, die die Bakterien kontrollieren sollen, welche an der Geruchsentstehung beteiligt sind.

Bei der Verwendung von antimikrobiellen Stoffen in Zubereitungen ist insbesondere ihre Veträglichkeit, aber auch ihre Formulierbarkeit (d.h. Löslichkeit, Stabiltät etc.) in den entsprechenden Produkten (z.B. Shampoos, Cremes, Deodorants) von großer Bedeutung. Insbesondere in der Kosmetik sind diese Eigenschaften essentiell. So ist es beispielsweise besonders erstrebenswert, dass die Inhaltsstoffe bei Normaldruck zwischen -5°C und 40°C in flüssigem Aggregatzustand vorliegen.
Ziel der vorliegenden Erfindung ist daher die Bereitstellung neuer Inhaltsstoffe mit antimikrobieller Wirkung, die die oben genannten vorteilhaften Eigenschaften aufweisen.

Überraschend wurde nun gefunden, dass bestimmte Cyclohexanoletherderivate die oben genannten Eigenschaften bei gleichzeitiger hervorragender antimikrobieller Wirkung aufweisen.
Im Stand der Technik sind spezielle Cyclohexanolderivate, sowie deren Verwendung in der Kosmetik, bekannt:
WO 2009/087242 A2 beschreibt die Verwendung von trans-4-tert-butyl-Cyclohexanol zur Reduktion von Hautirritationen.
WO 94/10117 offenbart die Verwendung von Cyclohexanolderivaten zur Erzeugung eines kühlenden Effekts auf der Haut.
US 2006/0045892 A1 offenbart Cyclohexanolderivate zur Verbesserung des Hauterscheinungsbildes durch Reduktion von Hautröte und Hautirritationen.
WO 2011/050871 offenbart die antibakteriellen, antimycotischen und antiviralen Menthylethers (bevorzugt Glycerolether) und die enthaltenden kosmetischen und dermatologischen Zubereitungen. JP 2007 291049 A beschreibt die Verwendung von den Glycerolcyclohexyletherderivaten als antimikrobielle Wirkstoffe vor allem in kosmetischen Formulierungen und ferner in Haushaltprodukten, Lebensmittelverpackungen und Kunststoffen. JP 2008050271 A beschreibt das antiseptische Make-up enthaltend Diethyleneglycol- oder Triethyleneglycolalkylcyclohexyletherderivaten. JP 2004 059536 A offenbart antimikrobiellen Fettalkoholcyclohexylethers. US 2003/232893 A1 offenbart Zusammensetzungen enthaltend Cyclohexanolderivate und ihre Verwendung zur Behandlung von *Rosacea* und *Akne vulgaris.* GB 1 315 626 A offenbart kosmetische Zubereitungen enthaltend 3-(2-Hydroxy-n-propyloxy)-p-menthane and 3-(2-Hydroxyethoxy)-p-menthane als Kälterezeptorstimulant. EP 2 2014 273 A offenbart die Verwendung von Menthylmethylether in Deodoranten und Antiperspiranten um Körpergeruch zu überdecken.

Ein erster Gegenstand der vorliegenden Erfindung ist daher die nicht-therapeutische Verwendung mindestens einer Verbindung der Formel I 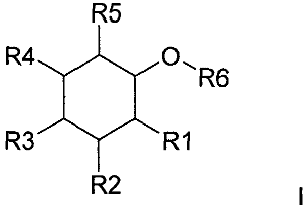 worin R1, R2, R4 und R5 unabhängig voneinander stehen für einen Rest ausgewählt aus H und t-Butyl und R3 steht für einen Rest ausgewählt aus
- H, OH, OCOCH₃, O(CH₂CH₂O)ₙH, wobei n = 1 bis 20, Halogen,
- geradkettige oder verzweigte Alkylgruppe mit 1 bis 20 C-Atomen, welche optional mit ein oder mehreren OH-Gruppen substituiert sein kann, und/ oder durch eine oder mehrere Gruppen ausgewählt aus -O-, -(C=O)-, -(CO)O- und Cyclohexyl unterbrochen sein kann,
- geradkettige oder verzweigte O-Alkylgruppe mit 1 bis 20 C-Atomen,
und worin R6 steht für einen Rest ausgewählt aus CH₃, CH₂CH(CH₃)OH und CH₂CH₂OH, als antimikrobieller Wirkstoff.

Ein weiterer Gegenstand der Erfindung sind die Verbindungen der Formel I, wie vorher definiert, zur Verwendung als antimikrobieller Wirkstoff in pharmazeutischen Formulierungen und/oder Medizinprodukten.

Unter einem antimikrobiellen Wirkstoff versteht man erfindungsgemäß eine Substanz, die das Wachstum von Mikroorganismen reduziert, oder die Mikroorganismen abtötet oder inaktiviert.

Die erfindungsgemäßen Verbindungen der Formel I können verwendet werden, um das Wachstum und die Vermehrung der Mikroorganismen zu hemmen. Unter Mikroorganismen werden beispielsweise Bakterien (grampositive und gram-negative Bakterien), Hefen, Pilze oder Viren verstanden. Beispiele von Mikroorganismen sind Mikroorganismen ausgewählt aus

*Staphylococcus, Micrococcus, Escherichia, Enterobacter, Pseudomonas, Bacillus, Salmonella, Serratia, Shigella, Porphyromonas, Prevotella, Wolinella, Campylobacter, Propionibacterium, Streptococcus, Corynebacterium, Treponema, Fusobacterium, Bifidobacterium, Lactobacillus, Actinomyces, Candida, Malassezia, Aspergillus, Herpes simplex 1 und 2.*

Im Speziellen sind erfindungsgemäße Verbindungen antimikrobiell wirksam gegen *Staphylococcus epidermidis, Staphylococcus aureus, Corynebacterium xerosis, Malassezia furfur, Propionibacterium acnes, Pseudomonas aeruginosa, Salmonella enteric, Serratia marcescens, Escherichia coli, Enterobacter gergovia, Aspergillus niger, Aspergillus brasiliensis* und *Candida albicans.*

Die antimikrobiellen Verbindungen der Formel I zeichnen sich durch eine gute antimikrobielle Aktivität aus, das bedeutet, dass die Zahl der Keime in einem Medium reproduzierbar reduziert werden kann oder die Keimvermehrung unterdrückt wird (siehe Beispiele).

Die erfindungsgemäßen antimikrobiellen Wirkstoffe können in einer Vielzahl von Formulierungen oder Anwendungen zum Einsatz kommen, wie beispielsweise kosmetische/ pharmazeutische Formulierungen, Medizinprodukte, Lebensmittel, Haushaltsprodukte, Kunststoffen, Plastisolen, Papier und/ oder Farben. Im Speziellen kann es sich zum Beispiel um antimikrobielle Reinigungsprodukte, Seifen, Desinfektionsmittel, Prothesen oder Knochenzement mit antimikrobieller Aktivität, Zahnfüllungen und -prothesen, Zahn- und Mundpflegeprodukte, Körperpflegeprodukte (Cremes, Shampoos, Lotionen, Waschprodukte, Deodorants, Antitranspirantien/ Antiperspirantien, antimikrobielle Handspülungen etc.), Hygieneartikel, Küchen- und Badezimmerartikel, Geschirrspülprodukte oder Lebensmittel und Getränke handeln.

Die Verbindungen der Formel I können vorteilhaft zur Konservierungsverbesserung eingesetzt werden.

Vorteilhaft ist beispielsweise die nicht-therapeutische Verwendung der erfindungsgemäßen antimikrobiellen Wirkstoffe in Zahn- oder Mundpflegeprodukten. Diese Wirkstoffe sind auch geeignet zur Behandlung oder Prophylaxe von Plaque, Karies oder Mundgeruch, ausgelöst durch Mikroorganismen wie *Streptococcus sobrinus, Streptococcus mutans, Streptococcus gordonii, Streptococcus salivaris, Streptococcus sanguis, Actinomyces, Lactobacilli, Fusobacterium, Veillonella, Treponema. denticola, Porphyromonas. gingivalis, Bacteroides* oder *Peptostreptococcus.*

Zudem können erfindungsgemäße Stoffe gegen Nagelpilz verursachende Dermatophyten wirksamkeitsunterstützend sein. Zu den Dermatophyten zählen z. B. *Trichophyton spp. (Trichophyton rubrum, Trichophyton mentagrophytes)* oder *Epidermophyton floccosum.*

Als multifunktionelle Stoffe eignen sich die Verbindungen der Formel I zur Verwendung als Antioxidantien oder duftgebende Geruchsstoffe zur Desodorierung und Maskierung unerwünschter Eigengerüche der Inhaltsstoffe in Formulierungen. Insbesondere ist in dieser Hinsicht eine Kombination mit weiteren Antioxidantien und Duftstoffen denkbar. Dies umfaßt z.B. alle Duftstoffe wie beschrieben in "S. Arctander, Perfume and Flavor Materials, Vol. I and II, Montclair, N.J., 1969, Selbstverlag" oder in "K. Bauer, D. Garbe and H. Surburg, Common Fragrance and Flavor Materials, 4th Ed., Wiley-VCH, Weinheim 2001" oder wie beschrieben in US7354893 B2, insbesondere beschrieben in US 7354893, Spalte 2, Zeilen 37 bis 67.

Erfindungsgemäße Stoffe eignen sich auf Grund ihrer Stoffeigenschaften hervorrragend zur Einarbeitung in Emulsionen und tensidische Zubereitungen wie z,.B. Reinigungsmittel und sog. Rinse-off-Zubereitungen wie z.B. Duschgele. Erfindungsgemäße Stoffe liegen z.B. bei Raumtemperatur in flüssiger Form vor und eignen sich zum einen als Lösungsmittel für Feststoffe, zum anderen zeigen sie Emollienteigenschaften mit gutem Spreitverhalten, welches ein angenehmes Hautgefühl der Formulierung bewirkt.

Ferner eignen sich erfindungsgemäße Stoffe als Mittel zur Erhöhung der Hautfeuchtigkeit insbesondere in synergistischer Kombination mit weiteren Hautfeuchthaltemitteln wie z.B. Glycerin, Glycerinderivate, Hyaluronsäure, Harnstoff, Harnstoffderivate, Ectoin, Milchsäure und Lactate, Collagen, AHAs. Beispielhafte Hautfeuchthaltemittel sind auf Seite 27, Zeile 4 bis Seite 28 Zeile 17 der WO 2009/098139 beschrieben.

Sie können ferner adstringierende, hautkühlende, antistatische oder haarkonditionierende Eigenschaften aufweisen. Die Verbindungen der Formel I eignen sich als Additive für die Hautpflege. Hierzu gehören Anti-Agingwirkung, Anti-Irritationswirkung und Antiinflammatorische Wirkung. Unerwünschte Hautrötung lässt sich somit reduzieren. Erfindungsgemäße Stoffe eignen sich zur Behandlung von bzw, zur Vorbeugung gegen Störungen des natürlichen Gleichgewichts der Hautflora, wie z.B. bei Neurodermitis.

Erfindungsgemäße Stoffe können beispielsweise Verwendung finden zur Verbesserung der Hautbarrierefunktion, insbesondere in synergistischer Kombination mit weiteren Wirkstoffen in diesem Bereich, wie z.B. Lanolin, Shea butter, Phospholipide, Cholesterin und Cholesterinderivate, Phytosterole, essentielle Fettsäuren wie Linolsäure und Linolensäure, omega-3 ungesättigte Öle, Ceramide, wie z.B. Typ 2 oder 3 Ceramide, Sphigosine, wie z.B. Salicyloyl sphingosine oder Aminosäure wie Serin oder Arginin. Beispielhafte Wirkstoffe, die die Hautbarrierefunktion verbessern sind auf Seite 30, Zeile 6 bis Seite 31, Zeile 10 der WO 2009/098139 beschrieben.

Es ist ferner denkbar, daß erfindungsgemäße Stoffe Positiveffekte in Mitteln zur Pigmentierungskontrolle, wie z.B. chemisch oder biologisch wirkenden hautaufhellenden oder chemisch oder biologisch wirkenden hautbräunenden Wirkstoffen, zeigen, d.h. unterstützend die Hautfarbe verdunkeln oder aufhellen. Im Falle der Verwendung zur Hautaufhellung ist insbesondere die Kombination mit weiteren hautaufhellenden Wirkstoffen bevorzugt. Dazu gehören z.B. Vitamin C und Vitamin C Derivate wie 2-O-Vitamin C Glucosid, 2-O-Vitamin C Phosphat, 2-O-, bzw. 3-O-Ethyl Vitamin C oder 6-O-p-Methoxycinnamoylascorbinsäure, alpha und beta Arbutin, Ferulasäure, Lucinol und Lucinolderivate, Kojic acid, Resorcinol und Resorcinolderivate, Tranexamsäure und ihre Derivate, Gentisinsäure und ihre Derivate, Liponsäure, Ellaginsäure, Vitamin B3 und Vitamin B3 Derivate, Extrakte wie z.B. Maulbeerextrakte. Beispielhafte hautaufhellende Wirkstoffe sind auf Seite 31, Zeile 14 bis Seite 32, Zeile 7 der WO 2009/098139 beschrieben.
Ebenso denkbar sind Mittel zur Hautaufhellung enthaltend erfindungsgemäße Stoffe in Kombination mit Stoffen wie in WO 2007/121845 offenbart, insbesondere der Verbindungen der Ansprüche 12 und 13 der WO 2007/121845.

Weitere Funktionseigenschaften, die die erfindungsgemäßen Substanzen beinhalten können, bzw. die von ihnen in Kombination mit spezifischen Wirkstoffen unterstützt bzw. verbessert werden können, umfassen z.B. die in WO2009/098139 aufgeführten Substanzen.

Insbesondere in der Kombination mit traditionellen Konservierungsmitteln lassen sich Verbesserungen im Konservierungsergebnis erzielen. Die Wirkung kosmetischer Alkohole, wie z.B. Glycole, kann durch die Stoffe verstärkt werden.

In Lichtschutzformulierungen eignen sich erfindungsgemäße Stoffe hervorragend, um Schutzfaktoren zu erhöhen (Sonnenschutzfaktor, UVA-Schutzfaktoren wie PPD [persistent pigment darkening], Faktoren, die den Schutz gegen Infrarot-, oder sichtbares Licht ausdrücken).

Weitere exemplarische Wirkungen umfassen Antiglykationswirkung, Dermorelaxierende Wirkung, Aktivierung hauteigener Makromoleküle wie z.B. Aktivierung von Kollagen und Elastin sowie deren Schutz vor Abbau, Aktivierung der Fibroblasten- oder Keratinozytenproliferation, Inhibierung der NO-Synthase, sebumregulierende Wirkung, zellenergiestoffwechselstimulierende Wirkung, insbesondere in Kombination mit Salzen des Mangans, Zinks, Kupfers, Magnesiums und beta-Glucan, hautstraffende Wirkung, Anticellulitewirkung, insbesondere in Kombination mit Xanthinen wie z.B. Koffein, fettrestrukturierende Wirkung, wie z.B. lipolytische Wirkung (Slimmingwirkung), antiinflammatorische Wirkung, z.B. in Kombination mit Hydrocortison und Folsäure bzw. deren Derivaten,
Ferner ist es denkbar, daß erfindungsgemäße Substanzen präventiv der Hautalterung, auch der lichtbedingten Hautalterung, entgegenwirken, indem sie z.B. Matrixmetalloproteinasen (MMPs) inhibieren oder zum DNA-Schutz beitragen. In einer weiteren Applikation tragen die Substanzen zur Wundheilung bei.

Erfindungsgemäße Stoffe können ferner als sog. Netzmittel fungieren und so durch Herabsetzung der Oberflächenspannung am Bakterium das Eindringen von Konservierungsmitteln erleichtern.
Weiterhin können die erfindungsgemäßen Stoffe auch als Weichmacher fungieren, z.B. bei der Herstellung von Plastisolen, Kunststoffen, Gummi, Lacken und Klebstoffen, insbesondere zu deren phthalatfreien Herstellung.

Ein weiterer Gegenstand der vorliegenden Erfindung ist die Verwendung der Verbindungen der Formel I, wie in Anspruch 1 definiert, als Deodorant- oder Antitranspirantwirkstoff.

Insbesondere eignen sich die Verbindungen der Formel I als Anti-Aknewirkstoff zur Behandlung oder Prophylaxe von Akne, welche durch Mikroorganismen wie *Propionibacterium acnes, Propionibacterium granulosum* oder *Staphylococcus epidermidis* ausgelöst wird. Geeignete Formulierungen hierfür sind weiter unten beschrieben.

Weiterhin eignen sich die Verbindungen der Formel I als Antischuppen-Wirkstoff sowohl zur Behandlung als auch zur Prophylaxe. Geeignete Formulierungen, beispielsweise Shampoos, sind ebenfalls weiter unten beschrieben.

Vorteilhaft ist auch die Verwendung als Wirkstoff in Deodorants oder Antitranspirantien. Zum Einen haben einige der erfindungsgemäßen Verbindungen einen Eigengeruch, welcher unangenehme Gerüche überdecken und so desodorierend wirken kann. Ein weiterer Vorteil ist, dass die Verbindungen die Hautfeuchtigkeit erhöhen und sich damit vorteilhaft auf das Erscheinungsbild der Haut auswirken (z.B. hautglättende Wirkung).
Die Verwendung in Deodorants oder Antitranspirantien ist weiterhin vorteilhaft, da die Verbindungen der Formel I eine antimikrobielle Wirkung gegen die Bakterien aufweisen, die für die Zersetzung des Schweißes und damit für die Entstehung des Geruchs verantwortlich sind. Besonders von Vorteil ist hierbei, dass die Verbindungen je nach Testkeim bakteriostatisch oder bakterizid wirken können. Eine bakteriostatische Wirkung wird erzielt, wenn die Vermehrung eines Bakteriums zwar gehemmt wird, wodurch die Geruchsentstehung unterdrückt wird, jedoch das Bakterium nicht abgetötet wird. Dadurch kann die natürliche Hautflora vorteilhaft aufrechterhalten werden.

Insbesondere weisen die Verbindungen der Formel I eine bakteriostatische Wirkung gegen *Staphylococcus epidermidis* auf.
Mögliche Formulierungen für Deodorants oder Antitranspirantien sind weiter unten beschrieben.

Bevorzugt ist die Verwendung kosmetisch. So ist eine nicht-therapeutische Verwendung der o.g. Verbindungen oder deren Zubereitungen zur Vorbeugung von unerwünschter Veränderungen des Hautbildes, wie sie bei Akne auftritt, möglich.
Die Verbindungen der Formel I weisen eine unerwartet gute antimikrobielle Wirkung auf, wie in den Beispielen dargelegt wird. Weiterhin sind die Verbindungen der Formel I vorteilhaft in Formulierungen verwendbar, da sie auf Grund ihrer guten Löslichkeiten gut in die entsprechenden Formulierungen eingearbeitet werden können.
Erfindungsgemäß werden von den Verbindungen der Formeln I alle möglichen Ringkonfigurationsisomere umfasst, d.h. sowohl cis-, als auch trans-Isomere sind denkbar.
Erfindungsgemäß werden von den Verbindungen der Formeln I auch Verbindungen umfasst, in denen ein oder mehrere Atome durch andere Isotope ersetzt sind, insbesondere Verbindungen, in denen H-Atome durch Deuterium (D) ersetzt sind.

In den Verbindungen der Formel I steht R6 für einen Rest ausgewählt aus CH₃, CH₂CH(CH₃)OHund CH₂CH₂OH.

R1 steht für einen Rest ausgewählt aus H und t-Butyl.

R2 in Formel I steht für einen Rest ausgewählt aus H und t-Butyl.

R3 in Formel I steht bevorzugt für einen Rest ausgewählt aus
- H, OH, O(CH₂CH₂O)ₙH, wobei n = 1 bis 2,
- geradkettige oder verzweigte Alkylgruppe mit 1 bis 12 C-Atomen, welche optional mit ein oder mehreren OH-Gruppen substituiert sein kann, und/ oder durch eine oder mehrere Gruppen ausgewählt aus -O-, -(C=O)-, -(CO)O- und Cyclohexyl unterbrochen sein kann,
- geradkettige oder verzweigte O-Alkylgruppe mit 1 bis 12 C-Atomen. Besonders bevorzugt steht R3 für einen Rest ausgewählt aus
- H, OH, O(CH₂CH₂O)ₙH, wobei n = 1 bis 2,
- geradkettige oder verzweigte Alkylgruppe mit 1 bis 6 C-Atomen, welche optional mit ein oder mehreren OH-Gruppen substituiert sein kann,
- geradkettige oder verzweigte O-Alkylgruppe mit 1 bis 6 C-Atomen. Ganz besonders bevorzugt steht R3 für einen Rest ausgewählt aus H, OH, OCH₂CH₂OH, OCH₃ und CH₂OH.

R4 in Formel I steht für einen Rest ausgewählt aus H und t-Butyl. In einer ganz besonders bevorzugten Ausführungsform steht R4 für H.

R5 in Formel steht für einen Rest ausgewählt aus H und t-Butyl. In einer ganz besonders bevorzugten Ausführungsform steht R5 für H. In einer Ausführungsform steht R3 für einen Rest ausgewählt aus - H, OH, O(CH₂CH₂O)ₙH, wobei n = 1 bis 2, - geradkettige oder verzweigte Alkylgruppe mit 1 bis 12 C-Atomen, welche optional mit ein oder mehreren OH-Gruppen substituiert sein kann, und/ oder durch eine oder mehrere Gruppen ausgewählt aus -O-, -(C=O)-, -(CO)O- und Cyclohexyl unterbrochen sein kann, - geradkettige oder verzweigte O-Alkylgruppe mit 1 bis 12 C-Atomen.

Insbesondere bevorzugt ist die Verwendung einer oder mehrerer Verbindungen ausgewählt aus den Verbindungen der Formeln I-1 bis I-9:

| | | | |
|---|---|---|---|
| I-1 | 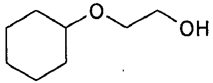 | I-2 | 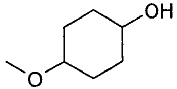 |
| I-3 | 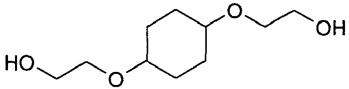 | I-4 | 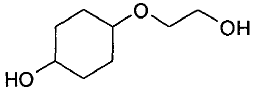 |
| I-5 | 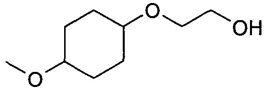 | I-6 | 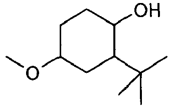 |
| I-7 | 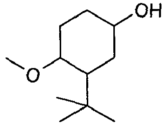 | I-8 | 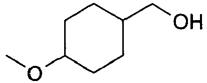 |
| I-9 | 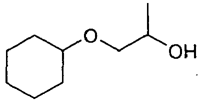 | | |

Ganz besonders bevorzugt ist die Verwendung der Verbindung der Formel I-1, I-2, I-3 und/oder I-9 (CAS 5334-13-4).

Weitere erfindungsgemäß verwendbare Verbindungen sind zum Beispiel die Verbindungen der Formel I-10 bis I-16

| | | | |
|---|---|---|---|
| I-10 | 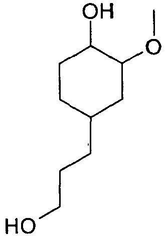 | I-11 | 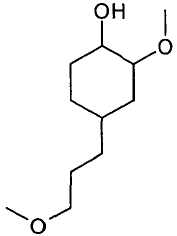 |
| I-12 | 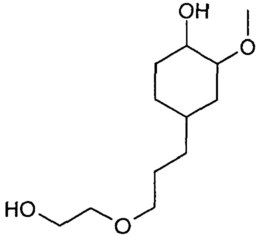 | I-13 | 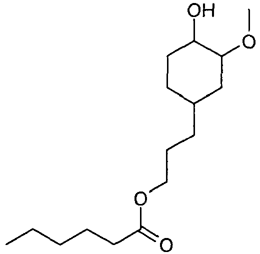 |
| I-14 | 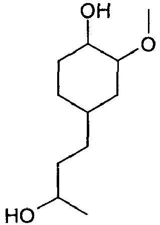 | I-15 | 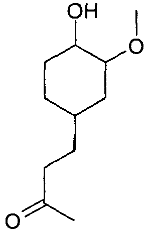 |
| I-16 | 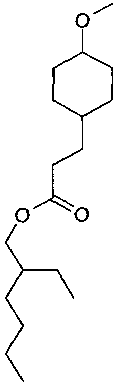 | | |

oder auch die Verbindungen der Formel I-17 bis I-20:

| | | | |
|---|---|---|---|
| I-17 | 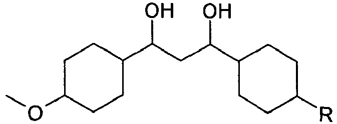 | I-18 | 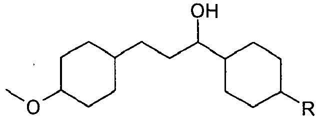 |
| I-19 | 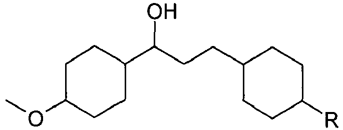 | I-20 | 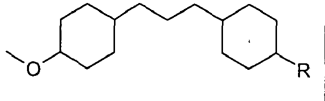 |

wobei R in den Formeln I-17 bis I-20 für H, Methoxy, t-Butyl oder Isopropyl steht.

Im Sinne der vorliegenden Erfindung handelt es sich bei einer geradkettigen oder verzweigten Alkylgruppe mit 1 bis 6 C-Atomen beispielsweise um Methyl, Ethyl, Isopropyl, Propyl, Butyl, sek.-Butyl oder tert.-Butyl, Pentyl, Isopentyl, 1-, 2- oder 3-Methylbutyl, 1,1-, 1,2- oder 2,2-Dimethylpropyl, 1-Ethylpropyl, 1-Ethyl-1-methylpropyl, 1-Ethyl-2-methylpropyl, 1,1,2- oder 1,2,2-Trimethylpropyl, 1,1-, 1,2-, 1,3-, 2,2-, 2,3-oder 3,3-Dimethylbutyl, 1- oder 2-Ethylbutyl, 1-, 2-, 3- oder 4-Methylpentyl oder Hexyl.
Bei einer Alkylgruppe mit 1 bis 12 C-Atomen kann es sich neben den oben gelisteten Resten beispielsweise auch um Heptyl, 1-Ethyl-pentyl, Octyl, 1-Ethyl-hexyl, Nonyl, Decyl, Undecyl oder Dodecyl handeln.
Unter eine Alkylgruppe mit 1 bis 20 C-Atomen versteht man ferner Tridecyl, Tetradecyl, Pentadecyl, Hexadecyl, Heptadecyl, Octadecyl, Nonadecyl oder Eicosyl.

Analog handelt es sich bei einer O-Alkylgruppe um eine Alkylgruppe, wie oben definiert, die über ein Sauerstoffatom an die Verbindung der Formel I gebunden ist.

Halogen steht für ein Halogenrest, bevorzugt für einen Rest ausgewählt aus F, Cl, Br oder I.

Die Verbindungen der Formel I können mittels Hydrierung entsprechender aromatische Ausgangsprodukte nach dem Fachmann bekannten Methoden mit Wasserstoff und unter Verwendung eines geeigneten Ni-, Co-, Pt-, Pd-oder Rh-Katalysators hergestellt werden (siehe z.B. Alonso et al, 2008, Tetrahedron 64, 1847-1852 oder Bai et al, 2010, Catalysis Communications 12, 212-216).

Alternativ ist es erfindungsgemäß auch möglich, die Hydrierung mit Deuteriumgas (D₂) durchzuführen, um zu den entsprechenden deuterierten Substanzen zu gelangen.
Die Edukte, sowie die weiteren notwendigen Stoffe in der Synthese sind kommerziell erhältlich oder durch Synthesen zugänglich, die dem Fachmann aus der Literatur bekannt sind. Dabei bereitet es dem Fachmann keine Schwierigkeiten, die geeigneten Reaktionsbedingungen wie Lösungsmittel oder Temperatur auszuwählen.
Mögliche Edukte für die Substanzen I-10 bis I-15 finden sich zum Beispiel in WO 2012/080152 und WO 2012/080153. Mögliche Edukte für I-1 sind Phenoxyethanol von z.B. Lanxess sowie für I-9 Phenoxyisopropanol, wie z.B. von der Firma Parchem erhältlich.
Für I-16 eignet sich als Edukt Ethylhexylmethoxycinnamat, z.B. Eusolex 2292 der Firma Merck KGaA.
Typischerweise erfolgt die Reaktion bei Temperaturen unter 100°C und einem Druck kleiner 200 bar, z.B. bei 100 bar oder 5 bar.
Beispiele für mögliche Lösungsmittel sind 2-Propanol, Ethylacetat oder Tetrahydrofuran.
Die Reaktionszeit beträgt typischerweise mehrere Stunden, beispielsweise 1 bis 5 Stunden.
Ein weiterer Gegenstand der vorliegenden Erfindung ist auch eine Zubereitung enthaltend mindestens eine Verbindung der Formel I, wie zuvor definiert, und mindestens einen geeigneten Träger, dadurch gekennzeichnet, dass neben der mindestens einen Verbindung der Formel I mindestens ein weiterer Konservierungsstoff und/oder antimikrobieller Wirkstoff enthalten ist.

Bei der Zubereitung kann es sich beispielsweise um kosmetische/ pharmazeutische Formulierungen, Medizinprodukte, Lebensmittel, Haushaltsprodukte, Kunststoffe, Plastisole, Papier und/oder Farben handeln. Im Speziellen kann es sich zum Beispiel um antimikrobielle Reinigungsprodukte, Seifen, Desinfektionsmittel, Prothesen oder Knochenzement mit antimikrobieller Aktivität, Zahnfüllungen und - prothesen, Zahn- und Mundpflegeprodukte, Körperpflegeprodukte (Cremes, Shampoos, Lotionen, Waschprodukte, Deodorants, Antiperspirants, antimikrobielle Handwaschlotionen etc.), Hygieneartikel, Küchen- und Badezimmerartikel, Geschirrspülprodukte oder Lebensmittel und Getränke handeln.

In einer bevorzugten Ausführungsform ist die Zubereitung eine kosmetische und/ oder eine pharmazeutische Zubereitung. Besonders bevorzugt handelt es sich um eine kosmetische Zubereitung.

Bevorzugte Ausführungsformen der Reste R3 bis R5 der Verbindungen der Formel I sind hierbei wie zuvor beschrieben definiert. Bei den Zubereitungen handelt es sich dabei beispielsweise um topisch anwendbare Zubereitungen, beispielsweise kosmetische oder dermatologische Formulierungen oder Medizinprodukte. Die Zubereitungen enthalten in diesem Fall einen topisch kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.
Zubereitungen zur Anwendung im Mund enthalten einen Träger, der für diese Anwendungen geeignet ist.

Im Sinne der vorliegenden Erfindung wird neben dem Begriff Zubereitung gleichbedeutend auch der Begriff Mittel oder Formulierung verwendet.
Topisch anwendbar bedeutet im Sinne der Erfindung, dass die Zubereitung äußerlich einschließlich der Mundhöhle und örtlich angewendet wird, d.h. dass die Zubereitung geeignet sein muss, um beispielsweise auf die Haut aufgetragen werden zu können.

Die Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Die mindestens eine Verbindung der Formel I wird in den erfindungsgemäßen Zubereitungen typischerweise in Mengen von 0,01 bis 20 Gew.-%, bevorzugt in Mengen von 0,05 bis 10 Gew.-%, besonders bevorzugt in Mengen von 0,1 Gew.-% bis 5 Gew.-% und ganz besonders bevorzugt in Mengen von 0,5 bis 2 Gew.-%, bezogen auf die Gesamtmenge der Zubereitung, eingesetzt. Dabei bereitet es dem Fachmann keinerlei Schwierigkeiten die Mengen abhängig von der beabsichtigten Wirkung der Zubereitung entsprechend auszuwählen.

Bevorzugt handelt es sich bei der Zubereitung um ein Deodorant, ein Antitranspirant, ein Anti-Schuppen- oder Anti-Akne-Mittel oder eine antibakterielle Zubereitung, insbesondere zur Zahn- oder Mundpflege.

Anti-Akne-Mittel enthaltend die erfindungsgemäßen Verbindungen können in der Form von Seifen, Reiniger, Lösungen, Suspensionen, Emulsionen, Cremes, Gels, Pasten, Lotionen, Pudern, Ölen, Stiften oder Sprays vorliegen. Weitere mögliche Inhaltsstoffe in den Formulierungen sind weiter unten im Detail beschrieben.

Anti-Schuppen-Mittel enthaltend die erfindungsgegmäßen Verbindungen können beispielsweise in der Form eines Shampoos oder einer Spülung vorliegen, die vor oder nach dem Waschen, dem Färben oder dem Bleichen der Haare angewandt werden kann. Alternativ ist auch eine Formulierung möglich, in Form einer Lotion oder eines Gels zum Haarstyling, zur Haarbehandlung oder zur Haartrocknung, in Form eines Haarlacks, einer Formulierung für Dauerwelle, oder einer Formulierung zur Haarfärbung oder -bleichung. Eine solche kosmetische Formulierung kann eine Reihe von weiteren Inhaltsstoffen enthalten, wie oberflächenaktive Stoffe, Verdickungsmittel, Polymere, Weichmacher, Konservierungsstoffe, Schaumstabilisatoren, Elektrolyte, organische Lösungsmittel, Silikonderivate, Antifettwirksoffe, Farbstoffe oder Pigmente, die die Formulierung oder das Haar färben, oder andere Inhaltsstoffe, die gewöhnlich in Haarpflegeprodukten verwendet werden. Weitere Inhaltsstoffe werden im folgenden der Anmeldung detailliert beschrieben.

Deodorants oder Antitranspirantien/ Antiperspirantien können beispielsweise in der Form von Cremes, Gelen, Lotionen, Emulsionen, Deodorantstiften, -rollern, Sprays oder Pumpsprays vorliegen. Die Verbindungen der Formel I werden gewöhnlich mit einem für Deodorants oder Antitranspirantien geeigneten Trägermaterialien, Inhaltsstoffen und Wirkstoffen kombiniert.

Beispielhaft seien hier Kombinationen mit allen in WO 2011/131474 genannten Trägermaterialien, Inhaltsstoffen und Wirkstoffen genannt. Als besonders bevorzugt werden Kombinationen mit weiteren Deo-Adjuvantien erachtet, wie zum Beispiel aus den Gruppen der Silbersalze und/oder Silberkomplexe und/oder Mineralen vulkanischer Herkunft und/oder Zeolithen und/oder Alaun und/oder Haarwuchsinhibierenden Stoffen. Beispielhafte Silbersalze und/oder Silberkomplexe werden auf Seite 6, Zeile 7 bis Seite 10, Zeile 14 der WO 2011/131474 beschrieben. Mineralien vulkanischer Herkunft sind beispielhaft auf Seite 10, Zeile 15 bis Seite 11, Zeile 5 der WO 2011/131474 beschrieben. Zeolithe sind beispielhaft auf Seite 11, Zeile 7 bis Zeile 27 der WO 2011/131474 beschrieben. Salze vom Alaun-Typ sind beispielhaft auf Seite 11, Zeile 28 bis Seite 13, Zeile 17 der WO 2011/131474 beschrieben. Beispielhafte Haarwuchs inhibierende Substanzen sind auf Seite 13, Zeile 29 bis Seite 21, Zeile 11 der WO 2011/131474 beschrieben.

Beispiele für geeignete Trägermaterialien sind Glycerylstearat, Aluminiumchlorhydrat, Propylenglycol, Carbomer, Glycerin, Dicaprylether, Ethanol, Glycerylcocoat, Cylomethicone, Dimethicone, Dipropylenglycol, Stearylalcohol, Mineralöl, Phenyltrimethicone oder Natriumstearat.

In den oben genannten Formulierungen können die Verbindungen der Formel I vorteilhaft mit allen weiteren bekannten Konservierungsstoffen oder antimikrobiellen Wirkstoffen kombiniert werden. Dies kann vorteilhaft synergistische Effekte mit den Verbindungen der Formel I zur Folge haben. Beispiele für solche Konservierungsstoffe oder antimikrobielle Wirkstoffe sind Anissäure, Alkohol, Ammomiumbenzoat, Ammoniumpropionat, Benzoesäure, Benzoylperoxid, Bronopol, Butylparaben, Benzethoniumchlorid, Benzalkoniumchlorid, 5-Brom-5-nitro-1,3-dioxan, Benzylalkohol, Borsäure, Benzisothiazolinon, Benzotriazol, Benzylhemiformat, Benzylparaben, 2-Brom-2-Nitropropan-1,3-Diol, Butylbenzoat, Caprylylglycol, Chlorphenesin, Capryl/Capringlyceride, Caprylylglycol, Camellia Sinensis Blattextrakt, Candida Bombicola/glucose/methyl rapeseedate, Chlorxylenol, Chloracetamid, Chlorhexidin, Chlorbutanol, Calciumbenzoat, Calciumparaben, Calciumpropionat, Calciumsalicylat, Calciumsorbat, Captan, Chloramin T, Chlorhexidindiacetat, Chlorhexidindigluconat, Chlorhexidindithydrochlorid, Chloracetamin, p-Chlor-m-Cresol, Chlorophen, p-Chlorphenol, Chlorthymol, Climbazole, Citrus Grandis (Grapefruit) Fruchtextract, Citrus Grandis (Grapefruit) Samenextract, m-Cresol, o-Cresol, p-Cresol, gemischte Cresole, 1,2-Decandiol (INCI: Decylene Glycol), Diazolidinylharnstoff, Dichlorobenzylalcohol, Dimethyloxazolidin, DMDM Hydantoin, Dimethylhydroxmethylpyrazol, Dehydroessigsäure, Diazolidinylharnstoff, DEDM Hydantoin, DEDM Hydantoin Dilaurat, Dibrompropamidindiisothionat, Dimethylolethylenthioharnstoff, Dithiomethylbenzamid, DMDM Hydantoine, DMHF, Domiphenbromid, 7-Ethylbicyclooxazolidin, Ethylparaben, Ethylhexylglycerin, Ethanol, Ethylferulat, Farnesol, Formaldehyd, Ferulasäure, Fumarsäure, Glycerylcaprate, Glutaral, Glycerolformat, Glyoxal, Hexamidindiisethionat, Hexetidin, Hexamidin, Hexamidindiparaben, 1,2-Hexandiol, 1,6-Hexandiol, Hexamidinparaben, 4-Hydroxybenzoesäure, Hydroxymethyldioxazabicyclooctan, Imidazolidinylharnstoff, Imidiazolidinylharnstoff NF, Isobutylparaben, Isothiazolinon, lodpropynylbutylcarbamat, Isodecylparaben, Isopropylcresol, Isopropylparaben, Isopropylsorbat, Kaliumsorbat NF FCC, Kupferusnat, Kaliumbenzoat, Kaliumethylparaben, Kaliummethylparaben, Kaliumparaben, Kaliumphenoxid, Kalium o-Phenylphenat, Kaliumpropionat, Kaliumpropylparaben, Kaliumsalicylat, Kaliumsorbat, Methylparaben, Methylisothiazolinon, Methylchloroisothiazolinon, Methylbenzethoniumchloridphenol, Methyldibromglutaronitril, Methenammoniumchlorid, Methylbromglutaronitril, 2-Methyl 5-Cyclohexylpentanol, Magnesiumbenzoat, Magnesiumpropionat, Magnesiumsalicylat, MDM Hydantoin, MEA-Benzoat, MEA o-Phenylphenat, MEA-Salicylat, Methylchloristhiazolinon, Natriumbenzoat NF FCC, Natriumcaprylat, Natriumdehydroacetat, Natriumdehydroacetate FCC, Natriumhydroxymethylglycinat, Natriummethylparaben, Natriumpropylparaben, Natriumiodat, Niembaumsamenöl, Nisin, Natriumbenzoat, Natriumbutylparaben, Natrium-p-chlor-m-Cresol, Natriumethylparaben, Natriumformat, Natriumhydroxymethansulfonat, Natriumisobutylparaben, Natriumparaben, Natriumphenolsulfonat, Natriumphenoxid, Natrium-o-phenylphenat, Natriumpropionat, Natriumpropylparaben, Natriumpyrithion, Natriumsalicylat, Natriumsorbat, Octenidin, Ortholphenylphenol, Phenoxyethanol, Propylparaben, Polymethoxybicyclicoxazolidin, Pinus Pinaster Rindenextrakt, Poloxamer 188, PVP-lodine, Parabene, Pircotone olamine, Phenethylalkohol, Polyaminopropylbiguanid, Polyquarternium-42, PEG-5 DEDM Hydantoin, PEG-15 DEDM Hydantoin, PEG-5 Hydantoin Oleat, PEG-15 DEDM Hydantoin Stearat, Phenethylalcohol, Phenol, Phenoxyethylparaben, Phenoxyisopropanol, Phenylbenzoat, Phenyl Quecksilberacetat, Phenyl Quecksilberbenzoat, Phenyl Quecksilberborat, Phenyl Quecksilberbromid, Phenylquecksilberchlorid, Phenylparaben, o-Phenylphenol, Piroctone Olamine, Polyaminopropylbiguanidstearat, Propionsäure, Propylbenzoat, 1,2-Propylenglycol, 1,3-Propylenglycol, Quaternium-15, Quaternium-8, Quaternium-14, Rosmarinus officinalis Blattextrakt, Sorbinsäure NF FCC, Selenium disulphin, Sorbinsäure, Salicylsäure, Silberborsilicat, Silbermagnesiumaluminiumphosphat, Triclosan, di-alpha-Tocopherol, Tocopherolacetat, Thimersal, Triclocarban, TEA-Sorbat, Thimerosal, Usnic acid, Undecylenoyl PEG-5 Paraben, Vitis vinifera Samenextrakt, Teebaumöl, Wasserstoffperoxid, Zinkpyrithion, Zinkoxid, Zinkphenolsulphonat oder Kombinationen davon.

In den oben genannten Formulierungen können die Verbindungen der Formel I vorteilhaft mit einem oder mehreren Insektenschutzmitteln (Insect Repellants) kombiniert werden. Wichtige Insektenschutzmittel sind z.B. N,N-diethyl-m-toluamid (DEET), p-Menthan-3,8-diol (PMD) oder IR3535 (3-[N-Butyl-N-acetyl]-aminopropionic acid, ethyl ester).

Die Verbindungen der Formel I können auch mit Antibiotika kombiniert werden. Erfindungsgemäß können alle bekannten Antibiotika verwendet werden, zum Beispiel beta-Lactam, Vancomycin, Macrolide, Tetracycline, Quinolone, Fluorquinolone, nitrierte Verbindungen (wie Nitroxolin, Tilboquinol oder Nitrofurantoin), Aminoglycoside, Phenicole, Lincosamida, Synergistine, Fosfomycin, Fusidinsäure, Oxazolidinone, Rifamycine, Polymixyne, Gramicidine, Tyrocydine, Glycopeptide, Sulfonamide oder Trimethoprime.

Die erfindungsgemäßen Formulierungen können auch vorteilhaft mindestens ein Antioxidans enthalten.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.
Ebenso können Antioxidantien die Stabilität der in den Zubereitungen enthaltenen Verbindungen, inbesondere die Stabilität enthaltener erfindungsgemäßer Cyclohexanolether unterstützen.

Bevorzugte Zubereitungen enthalten neben einem oder mehreren Verbindungen der Formel I zusätzlich ein oder mehrere Antioxidantien zum Schutz der Ether gegen Autoxidation.

Erfindungsgemäß können Mischungen aus mindestens einer Verbindung der Formel I und mindestens einem Antioxidans auch in Form einer Vormischung vorliegen, welche als solche in die Zubereitung eingearbeitet wird.
Dementsprechend ist auch eine Zusammensetzung enthaltend mindestens eine Verbindung der Formel I, wie zuvor definiert, und mindestens ein Antioxidans Gegenstand der vorliegenden Erfindung. Geeignete Antioxidantien sind im Folgenden aufgeführt.
Bevorzugte Zusammensetzungen, welche als Vormischung dienen können, enthalten mindestens eine Verbindung der Formel I und mindestens ein Antioxidans im Gewichtsverhältnis von 99,9 : 0,1 bis 50,0 : 50,0.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA, Pentasodium ethylenediamin tetramethylen phosphonat und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Phytantriol, Coenzym Q10, Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selen¬methionin), Stilbene und deren Derivate (z.B. Stilbenoxid, trans-Stilbenoxid).

Geeignete Antioxidantien sind auch Verbindungen der Formeln A oder B 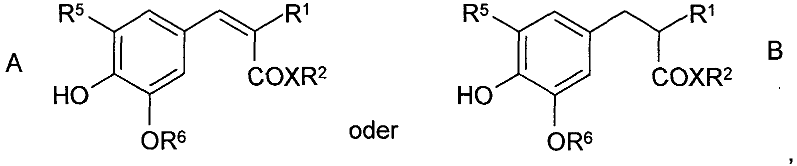 worin
- R¹: aus der Gruppe -C(O)CH₃, -CO₂R³, -C(O)NH₂ und -C(O)N(R⁴)₂ ausgewählt werden kann,
- X: O oder NH,
- R²: lineares oder verzweigtes Alkyl mit 1 bis 30 C-Atomen,
- R³: lineares oder verzweigtes Alkyl mit 1 bis 20 C-Atomen,
- R⁴: jeweils unabhängig voneinander H oder lineares oder verzweigtes Alkyl mit 1 bis 8 C-Atomen,
- R⁵: H, lineares oder verzweigtes Alkyl mit 1 bis 8 C-Atomen oder lineares oder verzweigtes Alkoxy mit 1 bis 8 C-Atomen und
- R⁶: lineares oder verzweigtes Alkyl mit 1 bis 8 C-Atomen bedeutet,
vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzyl)-malonsäure-bis-(2-ethylhexyl)ester (z.B. RonaCare® AP).

Mischungen von Antioxidantien sind ebenfalls zur Verwendung in den erfindungsgemäßen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L (+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydroxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Üblicherweise werden Antioxidantien in Mengen von bis zu 5 Gewichts-% in die Formulierungen eingearbeitet.

Formulierungen zur Mund- oder Zahnpflege können beispielsweise in Form einer Zahncreme, eines Mundwassers, eines Zahnpulvers, eines Kaugummis, einer Pastille, eines Mundsprays, Zahnseide, Zahnzement oder -farbe sein.

Ensprechende Formulierungen können weitere gewöhnliche Inhaltsstoffe enthalten, wie zum Beispiel Feuchthaltemittel, oberflächenaktive Mittel, Strukturbildner, Gelbildner, Schleifmittel, Fluoridquellen, Desensibilisierungsmittel, Geschmacksstoffe, Farbstoffe, Süßstoffe, Konservierungsstoffe, antimikrobielle Stoffe oder Mittel gegen Zahnbelag oder Zahnstein enthalten.

Geeignete Feuchthaltemittel zur Verwendung in Zahncremes sind beispielsweise Mehrwertige Alkohole, wie Xylitol, Sorbitol, Glycerin, Propylenglycol oder Polyethylenglycol. Mischungen von Glycerin und Sorbitol sind besonders geeignet. Ein Feuchthaltemittel trägt dazu bei, dass Zahncremeformulierungen bei Luftkontakt nicht austrocknen und dass das Mundgefühl der Creme (weiche Beschaffenheit, Fließfähigkeit, Süße) angenehm ist. Typischerweise sind diese Feuchthaltemittel in Mengen von 0-85 Gewichts-%, bevorzugt von 0-60 Gewichts-% in der Zubereitung enthalten.

Geeignete oberflächenaktive Stoffe in Zahncremes, Mundwässern etc. sind typischerweise wasserlösliche organische Verbindungen und können anionisch, nicht-ionisch, kationisch oder amphoter sein. Bevorzugt sollte ein angemessen stabiler oberflächenaktiver Stoff ausgewählt werden. Anionische oberflächenaktive Stoffe sind zum Beispiel wasserlösliche Salze von C₁₀₋₁₈ Alkylsulphaten (z.B. Natriumlaurylsulfat), wasserlösliche Salze von C₁₀₋₁₈ ethoxylierten Alkylsulphaten, wasserlösliche Salze von C₁₀₋₁₈ Alkylsarcosinaten, wasserlösliche Salze von sulfonierten Monoglyceriden von C₁₀₋₁₈ Fettsäuren (z.B. Natrium Kokosfettsäure Monoglyceridsulfonat), Alkylarylsulfonate (z.B. Natriumdodecylbenzensulfonat) und Natriumsalze des Kokosfettsäureamids von N-methyltaurin.
Nichtionische oberflächenaktive Stoffe, welche für Mundpflegemittel geeignet sind, sind beispielsweise die Kondensationsprodukte der Alkylenoxidgruppen mit aliphatischen oder alkylaromatischen Verbindungen, wie Polyethylenoxidekondensate von Alkylphenolen, Ethylenoxid/propylenoxid Copolymere (erhältlich unter dem Namen 'Pluronic'), Ethylenoxid/Ethylendiamin Copolymere, Ethylenoxidkondensate von aliphatischen Alkoholen, langkettige tertiäre Aminoxide, langkettige tertiäre Phosphinoxide, langkettige Dialkylsulfoxides und Mischungen davon. Alternativen hierzu sind ethoxylierte Sorbitanester, welche zum Beispiel über ICI unter dem Namen "Tween" erhältlich sind. Kationische oberflächenaktive Mittel sind typischerweise quaternäre Ammoniumverbindungen mit einer C₈₋₁₈ Alkylkette, wie zum Beispiel Lauryltrimethylammoniumchlorid, Cetyltrimethylammoniumbromid, Cetylpyridiniumchlorid, di-Isobutylphenoxyethoxyethyldimethylbenzylammoniumchlorid, Kokosfettsäurealkyltrimethylammoniumnitrit und Cetylpyridiniumfluorid. Auch geeignet sind Benzylammoniumchlorid, Benzyldimethylstearylammoniumchlorid und tertiäre Amine mit einer C₁₋₁₈ Kohlenwasserstoffgruppe und zwei (Poly)oxyethylengruppen. Amphoterische oberflächenaktive Mittel sind typischerweise aliphatische sekundäre und tertiäre Amine, wobei die aliphatischen Reste geradkettig oder verzweigt sein können und worin einer der aliphatischen Reste eine C₈₋₁₈ Gruppe ist und der andere Rest eine anionische hydrophile Gruppe enthält, zum Beispiel Sulfonat, Carboxylat, Sulfate, Phosphonat oder Phosphat.
Normalerweise wird das oberflächenaktive Mittel in einer Menge von 0-20 Gew.-%, bevorzugt 0-10 Gew.-% in die Mundpflegeformulierung eingearbeitet.

Strukturbildner können in Zahncremes oder Kaugummis notwendig sein, um die gewünschten strukturellen Eigenschaften und gewünschte "Mundgefühl" zu ermöglichen. Geeignete Stoffe sind zum Beispiel natürliche Gummis wie Tragantgummi, Xanthangummi, Karayagummi und Gummiarabicum, Algenderivate wie Perlmoos und Alginate, Smektiterde wie Bentonit oder Hectorit, Carboxyvinylpolymere und wasserlösliche Cellulosederivate wie Hydroxyethylcellulose und Natriumcarboxymethylcellulose. Verbesserte Strukturen können auch erreicht werden, wenn zum Beispiel colloidales Magnesiumaluminiumsilicat verwendet wird. Typischerweise ist der Strukturbildner in einer Menge von 0-5 Gew.-%, bevorzugt 0-3 Gew.-% in der Mundhygieneformulierung enthalten.

Schleifmittel sollten bevorzugt in der Lage sein, die Zähne zu putzen und/oder zu polieren, ohne dabei den Zahnschmelz oder das Dentin zu beschädigen. Meist werden sie in Zahncremes oder Zahnpulvern verwendet, jedoch können sie auch in Mundwässern etc. zum Einsatz kommen. Geeignete Schleifmittel sind beispielsweise Silikaschleifmittel, wie hydratisierte Silikate oder Silikagele, insbesondere Silicaxerogele (z.B. 'Syloid' erhältlich bei W. R. Grace and Company). Ebenso geeignet sind die Silikamaterialien erhältlich unter dem Namen 'Zeodent' von J. M. Huber Corporation, und Diateomeenerde wie 'Celite' erhältlich von Johns-Manville Corporation. Alternative Schleifmittel sind Alumina, unlösliche Metaphosphate wie unlösliches Natriummetaphosphat, Calciumcarbonat, Dicalciumphosphat (in Dihydrat und wasserfreie Formen), Calciumpyrophosphat, Polymethoxylate und spezielle partikuläre aushärtbare polymerisierte Harze, wie Melaminharnstoffe, Melaminformaldehyde, Harnstoffformaldehyde, Melaminharnstoffformaldehyde, vernetzte Epoxide, Melamine, Phenolharze, hochreine Cellulosen (z.B. 'Elcema' erhältlich von Degussa AG), und vernetzte Polyester. Typischerweise werden Schleifmittel in einer Menge von 0-80 Gew.-%, bevorzugt 0-60 Gew.-% in der Mundhygieneformulierung eingearbeitet.

Geeignete Fluoridquellen sind zum Beispiel Natriumfluorid, Zinkfluorid, Kaliumfluorid, Aluminiumfluorid, Lithiumfluorid, Natriummonofluorphosphat, Zinnfluorid, Ammoniumfluorid, Ammoniumbifluorid und Aminfluorid. Die Fluoridquellen sind bevorzugt in geeigneten Mengen enthalten, um bei der Verwendung ca. 50 ppm bis ca. 4,000 ppm Fluoridion bereitzustellen. Geeignete Desensibilisierungsmittel sind zum Beispiel Formaldehyd, Kaliumnitrat, Trikaliumcitrat, Kaliumchlorid und Strontiumchlorid, Strontiumacetat und Natriumcitrat.

Geschmacksstoffe können beispielsweise ausgewählt sein aus Ölen der Pfefferminze, der Grünen Minze, der Moosbeere, der Sassafraswurzel und der Nelke. Süßstoffe können auch verwendet werden, zum Beispiel D-tryptophan, Saccharin, Dextrose, Aspartam, Levulose, Acesulfam, Dihydrochalcone und Natriumcyclamat. Typischerweise sind alle diese Geschmacksstoffe in Mengen von 0-5 Gew.-%, bevorzugt von 0-2 Gew.-% enthalten. Farbstoffe und Pigmente können hinzugefügt werden, um die Formulierung optisch attraktiver zu gestalten. Häufig wird Titandioxid verwendet um eine starke weiße Farbe zu erhalten.

Wie oben beschrieben können die erfindungsgemäßen Formulierungen zur Zahn- und Mundpflege auch ein oder mehrere weitere antimikrobielle Wirkstoffe enthalten. Geeignete Beispiele hierfür sind Zinksalze (wie Zinkcitrat), Cetylpyridiniumchlorid, bis-Biguanide (wie Chlorhexidin), aliphatische Amines, Bromchlorophene, Hexachlorophene, Salicylanilide, quaternäre Ammoniumverbindungen und Triclosan. Alternativ können enzymatische Systeme eingesetzt werden, z.B. kann ein System enthaltend Lactoperoxidase und Glucoseoxidase verwendet werden um antimikrobiell wirksame Mengen von Wasserstoffperoxid in Gegenwart von Glucose, Wasser und Sauerstoff zu generieren.

Die Formulierung kann auch Alkohol enthalten. Dies ist besonders vorteilhaft in Mundwässern.

Die erfindungsgemäßen Zubereitungen können auch neben den Verbindungen der Formel I und den oben beschriebenen Inhaltsstoffen, weitere Inhaltsstoffe enthalten. Im Folgenden werden weitere mögliche Inhaltsstoffe, insbesondere für kosmetische Zubereitungen, beschrieben.

Die erfindungsgemäßen Zubereitungen können zusätzlich mindestens einen UV-Filter enthalten.

Organische UV-Filter, sogenannte hydrophile oder lipophile Sonnenschutzfilter, sind im UVA-Bereich und/oder UVB-Bereich und/oder IR und/oder VIS-Bereich (Absorber) wirksam. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Symrise, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Symrise.

β,β-Diphenylacrylate Derivate: Octocrylene, z. B. vetrieben unter dem Namen "Eusolex® OCR" von der Firma Merck", "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50"; Benzophenone-3 oder Oxybenzone, z. B. vetrieben unter dem Namen "Uvinul M40";Benzophenone-4, z. B. vetrieben unter dem Namen "Uvinul MS40"; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Symrise.

Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. BASF.

Triazin Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine vertrieben als Tinosorb A2B von BASF, 2,2'-[6-(4-methoxyphenyl)-1,3,5-triazine-2,4-diyl]bis[5-(2-ethylhexyl)oxy]-phenol, vertrieben als Tinosorb S von BASF, N2,N4-bis[4-[5-(1,1-dimethylpropyl)-2-benzoxazolyl]phenyl]-N6-(2-ethylhexyl)-1,3,5-triazin-2,4,6-triamin vertrieben als Uvasorb K 2A von der Firma Sigma 3V.

Anthranilin Derivate: Menthyl anthranilate, z. B. vetrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

### Piperazinderivate wie beispielsweise die Verbindung

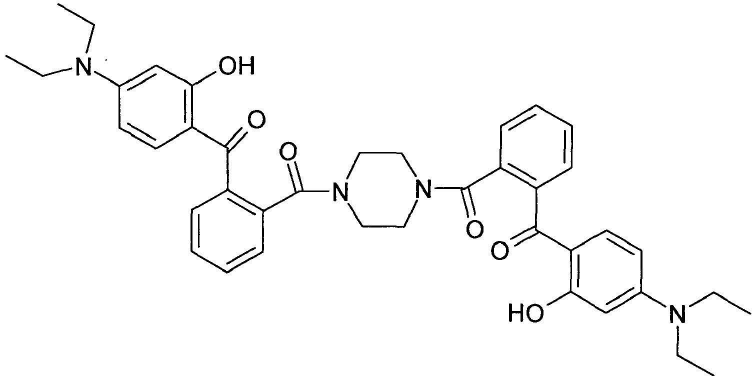

oder die UV-Filter der folgenden Strukturen 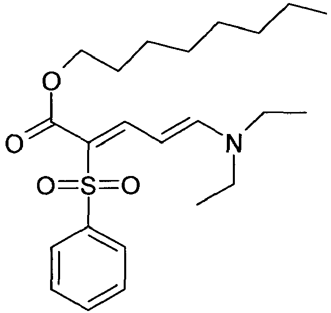 oder

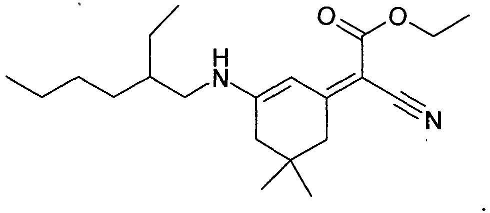

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

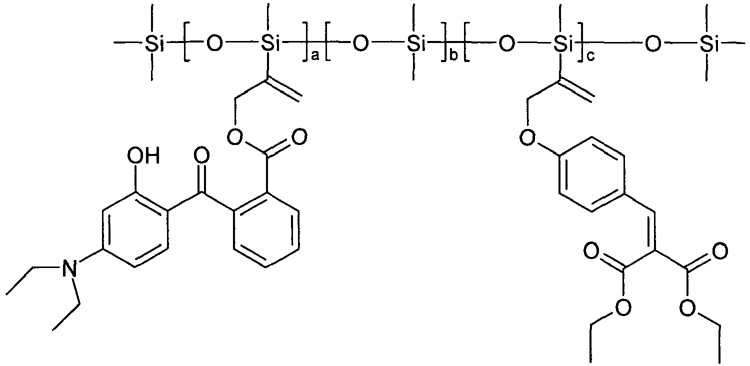

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl)imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Die Zubereitungen können neben den Verbindungen der Formel I sowie den gegebenenfalls organischen UV-Filtern, wie zuvor beschrieben, weitere anorganische UV-Filter, sogenannte partikuläre UV-Filter, enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex® T-2000, Eusolex®T-AQUA, Eusolex®T-AVO, Eusolex®T-OLEO), Zinkoxide (z.B. Sachtotec®), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53 64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexameta¬phosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   - "Zinc Oxide CS-5" der Fa. Toshibi (ZnO nachbehandelt mit polymethylhydrogenosiloxane)
   - Nanogard Zinc Oxide FN der Fa. Nanophase Technologies
   - "SPD-Z1" der Fa Shin-Etsu (ZnO nachbehandelt mit einem Silikongepfropften Acrylpolymer, dispergiert in Cyclodimethylsiloxane
   - "Escalol Z100" der Fa ISP (Aluminiumoxid nachbehandeltes ZnO dispergiert in einer ethylhexyl methoxycinnamate/PVP-hexadecene/ methicone copolymer Mischung)
   - "Fuji ZNO-SMS-10" der Fa. Fuji Pigment (ZnO nachbehandelt mit Siliciumdioxid und Polymethylsilesquioxan);
   - Unbehandeltes Ceroxide Mikropigment z.B. mit der Bezeichnung "Colloidal Cerium Oxide" der Fa Rhone Poulenc
   - Unbehandelte und/oder nachbehandelte Eisenoxide mit der Bezeichnung Nanogar der Fa. Arnaud.

Beispielhaft können auch Mischungen verschiedener Metalloxide, wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandeltem Titandioxid, Zinkoxid-Mischungen wie z.B . das Produkt UV-Titan M261 der Fa. Sachtleben verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen.

Dabei sind die Kapseln in erfindungsgemäß einzusetzenden Zubereitungen vorzugsweise in solchen Mengen enthalten, die gewährleisten, dass die verkapselten UV-Filter in den oben angegebenen
Gewichtsprozentverhältnissen in der Zubereitung vorliegen.

In den beschriebenen Zubereitungen, die erfindungsgemäß mindestens eine Verbindung der Formel I enthalten, können weiterhin auch Farbpigmente enthalten sein, wobei der Schichtaufbau der Pigmente nicht limitiert ist.

Vorzugsweise sollte das Farbpigment bei Einsatz von 0,5 bis 5 Gew.-% hautfarben oder bräunlich sein. Die Auswahl eines entsprechenden Pigments ist für den Fachmann geläufig.

Bevorzugte Zubereitungen können ebenfalls mindestens einen weiteren kosmetischen Wirkstoff enthalten, beispielsweise ausgewählt aus Anti-Ageing-, Anti-Falten-, weiteren Anti-Schuppen-, weiteren Anti-Akne-, Anti-Cellulite-Wirkstoffen, weiteren Deodorants, hautaufhellenden Wirkstoffen, Selbstbräunungssubstanzen oder Vitaminen.

Hinsichtlich der Anti-Aknewirkung sind synergistische Kombinationen mit weiteren Anti-Aknewirkstoffen, wie z.B. in WO2009/098139 auf Seite 47, Zeile 2 bis Seite 48, Zeile 27 und DE10324567 offenbart, denkbar. Exemplarische weitere Anti-Aknewirkstoffe sind Silberpartikel und Silbersalze wie Silberlactat und Silbercitrat, Azelainsäure, Ellaginsäure, Milchsäure, Glycolsäure, Salicylsäure, Glycyrrhizinsäure, Triclosan, Phenoxyethanol, Hexamidinisethionat, Ketoconazol, Peroxide wie Wasserstoffperoxid oder Benzoylperoxid, 3-Hydroxybenzoesäure, 4-hydroxybenzoesäure, Phytinsäure, Arachidonsäure, Caprylylglycol, Ethylhexylglycerin, Farnesol, Cetylpyridiniumsalze, 6-Trimethylpentyl-2-pyridon (Piroctone Olamine) und Lipohydroxysäure (LHA).

Hinsichtlich der Anti-Schuppenwirkung sind synergistische Kombinationen mit weiteren Anti-Schuppenwirkstoffen denkbar, wie z.B. Zinkpyrithion, Piroctone Olamine, Selendisulfid, Climbazole, Triclosan, Butylparaben, 1,3-Bis(hydroxymethyl)-5,5-dimethylimidazolidine-2,4-dione (DMDM Hydantoin), Fumarsäure, Methylchloroisothiazolinone oder Methylisothiazolinone (MIT),

In ihrer Verwendung in Desodorantien und Antitranspirantien lassen sich erfindungsgemäße Stoffe synergistisch mit weiteren Deo-Adjuvantien kombinieren. Hierzu sei auf die in WO2011/131474 aufgeführten Wirkstoffe verwiesen. Exemplarisch werden an dieser Stelle folgende kombinierbare Wirkstoffe aufgeführt: 2-Methyl 5-Cyclohexylpentanol, Aluminium Chlorohydrate, Ethylhexiglycerin, Farnesol, Aluminum Zirconium Tetrachlorohydrex GLY, Aluminium Chlorohydrate, Aluminium zirconium tetrachlorohydrate, Aluminum Sesquichlorohydrate
(Aluminumhydroxidchlorid), Triclosan, Aluminum tetrachloride, Zinc Ricinoleate, Aluminum Zirconium Pentachlorohydrate, Polyaminopropyl Biguanide Stearate, Benzyl Salicylate, Aluminum Sesquichlorohydrat, Zinc PCA (Zinksalz der Pyrrolidoncarbonsäure), Zinkgluconat Triethylcitrat, Aluminum Chloride, Aluminum Chlorohydrex Polyethylene Glycol Complex, Aluminum Chlorohydrex Propylene Glycol Complex, Aluminum Dichlorohydrate, Aluminum Dichlorohydrex Polyethylene Glycol Complex, Aluminum Dichlorohydrex Propylene Glycol Complex, Aluminum Sesquichlorohydrate, Aluminum Sesquichlorohydrex Polyethlene Glycol Complex, Aluminum Sesquichlorohydrex Propylene Glycol Complex, Aluminum Sulfate Buffered, Aluminum Zirconium Octachlorohydrate, Aluminum Zirconium Octachlorohydrex Glycine Complex, Aluminum Zirconium Pentachlorohydrate, Aluminum Zirconium Pentachlorohydrex Glycine Complex, Aluminum Zirconium Tetrachlorohydrate, Aluminum Zirconium Tetrachlorohydrex Glycine Complex, Aluminum Zirconium Trichlorhydrate, Aluminum Zirconium Trichlorohydrex Glycine Complex, Aluminum Zirconium Trichlorohydrex Glycine Complex, Aluminum Sulfate Buffered With Sodium Aluminum Lactate.

Die Zubereitungen können auch neben den Verbindungen der Formel I ein oder mehrere Anti-Ageing-Wirkstoffe enthalten. Geeignete Anti-Ageing Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin-Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myoinositol-phosphat (DIP), cyclisches 2,3-diphosphoglycerat (cDPG), 1,1-Diglycerin-Phosphat (DGP), β-Mannosylglycerat (Firoin), ß-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als Anti-Ageing Wirkstoffe Produkte der Firma Merck wie z.B. 5,7-Dihydroxy-2-methyl-chromon, vermarktet unter dem Handelsnamen RonaCare®Luremine, Ronacare®Isoquercetin, Ronacare®Tilirosid oderRonacare®Cyclopeptide 5 verwendet werden.

Die Zubereitungen können auch ein oder mehrere hautaufhellende Wirkstoffe oder synonym Depigmentierungswirkstoffe oder Melanogeneseinhibitoren enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin, Niacinamide, Azelainsäure, Elaginsäure, Maulbeerbaumextract, Magnesium-ascorbyl-phosphat, Süßholzwurzelextrakt, Emblica, Ascorbinsäure oder Rucinol.

Ferner können die erfindungsgemäßen Zubereitungen mindestens eine Selbstbräunungssubstanz als weiteren Inhaltsstoff enthalten.
Als vorteilhafte Selbstbräunungssubstanzen können unter anderem eingesetzt werden:
1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist 1,3-Dihydroxyaceton, Erythrulose oder deren Kombination. Bevorzugt ist die mindestens eine weitere Selbstbräunungssubstanz in der Zubereitung in einer Menge von 0,01 bis 20 Gew.-%, besonders bevorzugt in einer Menge von 0,5 bis 15 Gew.-% und ganz besonders bevorzugt in einer Menge von 1 bis 8 Gew.-%, bezogen auf die Gesamtmenge der Zubereitung, enthalten.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt.

Gegenstand der vorliegenden Erfindung ist auch ein Verfahren zur Herstellung einer Zubereitung, wie zuvor beschrieben, dadurch gekennzeichnet, dass mindestens eine Verbindung der Formel I mit einem geeigneten Träger und optional mit Hilfs- und oder Füllstoffen vermischt wird. Geeignete Trägerstoffe sowie Hilfs- oder Füllstoffe sind im nachfolgenden Teil ausführlich beschrieben.

Die genannten Bestandteile der Zubereitung können in der üblichen Weise eingearbeitet werden, mit Hilfe von Techniken, die dem Fachmann wohl bekannt sind.

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W) oder O/W/O, ein Gel, einen festen Stift, eine Salbe oder auch ein Aerosol darstellen. Bevorzugt werden Emulsionen. O/W-Emulsinen werden besonders bevorzugt. Emulsionen, W/O-Emulsionen und O/W-Emulsionen sind in üblicher Weise erhältlich.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole, Pflaster, Umschläge, Verbände und Sprays.

Bevorzugte Hilfsstoffe stammen aus der Gruppe der Konservierungsstoffe, Stabilisatoren, Lösungsvermittler, Färbemittel, Geruchsverbesserer. Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3 Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C Zahl, z.B. mit Isopropanol, Propylenglykol oder Glycerin, oder Ester von Fettalkoholen mit Alkansäuren niedriger C Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C Atomen und gesättigten und/oder ungesättigten, verzweigten und/oder unverzweigten Alkoholen einer Kettenlänge von 3 bis 30 C Atomen, aus der Gruppe der Ester aus aromatischen Carbonsäure und gesättigten und/oder ungesättigten, verzweigten und/oder unverzweigten Alkoholen einer Kettenlänge von 3 bis 30 C Atomen. Solche Esteröle können dann vorteilhaft gewählt werden aus der Gruppe Isopropylmyristat, Isopropylpalmitat, Isopropylstearat, Isopropyloleat, n Butylstearat, n-Hexyllaurat, n Decyloleat, Isooctylstearat, Isononylstearat, Isononylisononanoat, 2 Ethylhexylpalmitat, 2 Ethylhexyllaurat, 2 Hexaldecylstearat, 2 Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C Zahl, sowie deren Ether, vorzugsweise Ethanol, Isopropanol, Propylenglykol, Glycerin, Ethylenglykol, Ethylenglykolmonoethyl oder -monobutylether, Propylenglykolmonomethyl, -monoethyl oder -monobutylether, Diethylenglykolmonomethyl oder-monoethylether und analoge Produkte, ferner Alkohole niedriger C Zahl, z. B. Ethanol, Isopropanol, 1,2 Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen.

Es ist ferner von Vorteil, die Fettsäureethoxylate aus folgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat,
olyethylenglycol(21)glyceryllaurat, Polyethylenglycol(22)glyceryllaurat, Polyethylenglycol(23)glyceryllaurat,
Polyethylenglycol(6)glycerylcaprat/cprinat,
Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe
Polyethylenglycol(20)sorbitanmonolaurat,
Polyethylenglycol(20)sorbitanmonostearat,
Polyethylenglycol(20)sorbitanmonoisostearat,
Polyethylenglycol(20)sorbitanmonopalmitat,
Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C Atome, Diglycerinester gesättigter und/oder ungesättigter, verzweigter und/oder unverzweigter Alkancarbonsäuren einer Kettenlänge von 8 bis 24, insbesondere 12-18 C¬ Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Die Verbindungen der Formel I können erfindungsgemäß auch in Nahrungsmitteln verwendet werden. Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel I angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". Die Nahrungsmitteln können fest sein aber auch in flüssiger Form, also als Getränk, vorliegen.

Nahrungsmittel, die nach der vorliegenden Erfindung mit einer oder mehreren Verbindungen der Formel I angereichert werden können, sind beispielsweise Nahrungsmittel, die aus einer einzigen natürlichen Quelle stammen (z.B. Zucker, ungesüßter Saft, Korn, Getreide, Getreidesirup), Mischungen von derartigen Nahrungsmitteln (z.B. Multivitaminpräparate, Mineralstoffmischungen oder gezuckerter Saft) oder Nahrungsmittelzubereitungen (z.B. zubereitete Cerealien, Gebäck, Mischgetränke, Joghurt, Diätnahrungsmittel, kalorienarme Nahrungsmittel oder Tierfutter).
Die Nahrungsmittel, die nach der vorliegenden Erfindung mit einer oder mehreren Verbindungen der Formel I angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die mit einer oder mehreren Verbindungen der Formel I angereicherten erfindungsgemäßen Nahrungsmittel können mit Hilfe von Techniken hergestellt werden, die dem Fachmann wohl bekannt sind.

Ein weiterer Gegenstand der vorliegenden Erfindung sind auch Verbindungen der Formel I-4, I-5,I-6, I-11 bis I-16 oder I-17 bis I-20

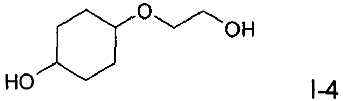

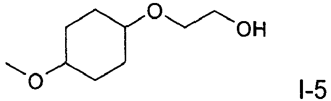

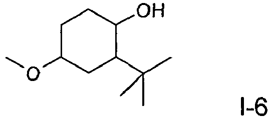

| | | | |
|---|---|---|---|
| I-11 | 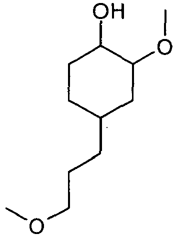 | I-12 | 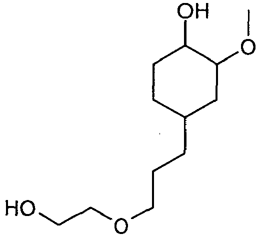 |
| I-13 | 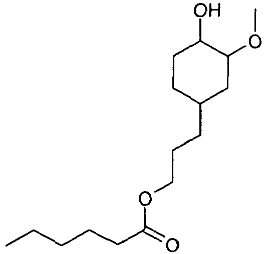 | I-14 | 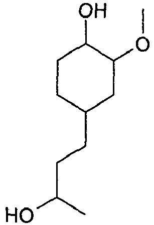 |
| I-15 | 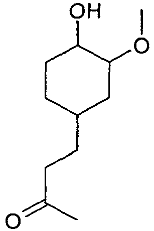 | I-16 | 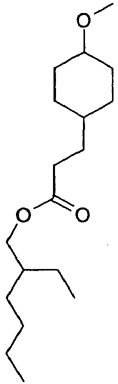 |
| | | | |
| I-17 | 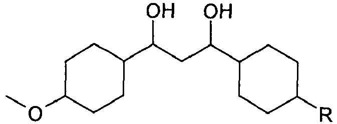 | I-18 | 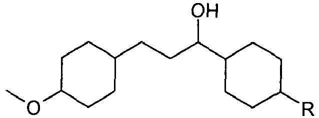 |
| I-19 | 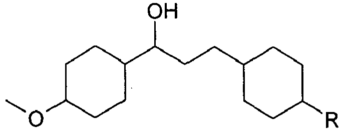 | I-20 | 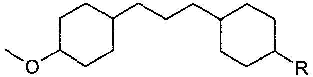 |

wobei R in den Formeln I-17 bis I-20 für H, Methoxy, t-Butyl oder Isopropyl steht und wobei I-20 mit R=H ausgeschlossen ist.

Bevorzugt sind die Verbindungen der Formeln I-4, I-5 und I-6.

Diese Verbindungen können wie zuvor beschrieben mittels Hydrierung entsprechender aromatischer Edukte unter Verwendung eines Katalysators nach dem Fachmann bekannten Methoden hergestellt werden.

Im folgenden wird die Erfindung anhand von Beispielen näher erläutert. Die Erfindung ist im gesamten beanspruchten Bereich ausführbar und nicht auf die hier genannten Beispiele beschränkt.

### Beispiele:

### Beispiel 1: Antimikrobielle Wirkung von I-2

Zur Probenvorbereitung wird die Untersuchungssubstanz I-2 zu 1,0% in einem Wasser-Ethanolgemisch (12% Ethanol) homogen dispergiert (= Probe). Es wird eine Suspension von *Staphylococcus epidermidis* (ATCC 12228) mit optischer Dichte von 1,0 in CASO-Bouillon (Merck 1.05459) 1:10000 mit CASO-Bouillon verdünnt. Man gibt zu 500µL Mikroorganismensuspension 500µL Probe und inkubiert bei 35°C für 24h bzw. 48h. Nach der Inkubation werden die Testansätze in 10er-Schritten bis -6 verdünnt und je 0,1 mL auf CASO-Agar (Merck 1.05458) ausplattiert und bei 35°C für 24h bebrütet.

Zur Bestimmung der Startkeimzahl werden 0,1 mL der verwendeten Bakteriensuspension auf CASO-Agar (Merck 1.05458) ausplattiert und für 24h bei 35°C inkubiert.

Anschließend werden die Kolonien gezählt und die Keimzahl pro mL im Vergleich zur Negativkontrolle (12% Ethanol in Wasser) ermittelt.

### (KBE = koloniebildende Einheit)

Ergebnis für Testsubstanz I-2:

| Testkeim | Startkeimzahl KBE/mL | Endkeimzahl KBE/mL |
|---|---|---|
| *Staphylococcus epidermidis* (ATCC 12228) | 5,8 x 10⁴ | 4,0 x 10⁴ |

Ergebnis für die Negativkontrolle:

| Testkeim | Startkeimzahl KBE/mL | Endkeimzahl KBE/mL |
|---|---|---|
| *Staphylococcus epidermidis* (ATCC 12228) | 5,8 x 10⁴ | 5,2 x 10⁷ |

### Beispiel 2: Antimikrobielle Wirkungen

### Durchführung in Anlehnung an Beispiel 1

Die angegebenen Konzentrationen sind die finalen Einsatzkonzentrationen. Zur Herstellung der Stammlösung werden die doppelte finale Konzentration im angegebenen Lösungsmittel hergestellt. Diese wird anschließend 1:2 mit der entsprechenden Keimsuspension verdünnt. Bei *Aspergillus brasiliensis* erfolgt die Keimzahlbestimmung abweichend nach 48h, bei Malassezia furfur nach 72h.

| Substanz | Konz. | Lösungsmittel | Keim | Keimzahl t₀ | Keimzahl t₂₄ₕ | Δlog |
|---|---|---|---|---|---|---|
| I-1 | 1,0% | H₂O | Propionibacterium acnes | 7,8 x 10⁵ | 7,0 x 10⁵ | -0,05 |
| - | - | H₂O | Propionibacterium acnes | 7,8 x 10⁵ | 3,9 x 10⁸ | +2,70 |
| I-3 | 0,5% | H₂O | Propionibacterium acnes | 3,4 x 10⁵ | 4,0 x 10⁷ | +2,07 |
| - | - | H₂O | Propionibacterium acnes | 3,4 x 10⁵ | 1,3 x 10⁸ | +2,58 |
| I-1 | 0,5% | H₂O | Corynebacterium xerosis | 1,0 x 10⁶ | 2,0 x 10⁶ | +0,30 |
| - | - | H₂O | Corynebacterium xerosis | 1,0 x 10⁶ | 6,0 x 10⁸ | +2,78 |
| | | | | | | |
| | | | | | | |
| I-1 | 0,8% | H₂O _{(12% Ethanol)} | Salmonella enterica | 1,3 x 10⁴ | 0 | -4,11 |
| - | - | H₂O _{(12% Ethanol)} | Salmonella enterica | 1,3 x 10⁴ | 4,9 x 10⁷ | +3,58 |
| I-1 | 0,5% | H₂O | Salmonella enterica | 1,0 x 10⁶ | 9,0 x 10³ | -2,05 |
| - | - | H₂O | Salmonella enterica | 1,0 x 10⁶ | 1,1 x 10⁹ | +3,04 |
| I-1 | 1,0% | H₂O | Serratia marcescens | 2,5 x 10⁵ | 0 | -5,40 |
| - | - | H₂O | Serratia marcescens | 2,5 x 10⁵ | 8,8 x 10⁸ | +3,55 |
| I-1 | 0,5% | H₂O | Serratia marcescens | 2,9 x 10⁵ | 1,7 x 10⁴ | -1,23 |
| - | - | H₂O | Serratia marcescens | 2,9 x 10⁵ | 9,7 x 10⁸ | +3,52 |
| I-1 | 1,0% | H₂O _{(12% Ethanol)} | Candida albicans | 6,0 x 10³ | 0 | -3,78 |
| - | - | H₂O _{(12% Ethanol)} | Candida albicans | 6,0 x 10³ | 5,0 x 10⁵ | +1,92 |
| | | | | | | |
| | | | | | | |
| I-1 | 0,8% | H₂O _{(12% Ethanol)} | Echerichia coli | 6,3 x 10⁴ | 0 | -4,80 |
| - | - | H₂O _{(12% ethanol)} | Echerichia coli | 6,3 x 10⁴ | 5,8 x 10⁷ | +2,96 |
| | | | | | | |
| | | | | | | |
| I-1 | 1,0% | H₂O _{(12% Ethanol)} | Staphylococcus aureus | 7,2 x 10⁴ | 2,5 x 10² | -2,46 |
| - | - | H₂O _{(12% Ethanol)} | Staphylococcus aureus | 7,2 x 10⁴ | 9,0 x 10⁷ | +3,10 |
| I-1 | 1,0% | H₂O _{(12% Ethanol)} | Aspergillus brasiliensis | 8,0 x 10² | 1,0 x 10² | -0,90 |
| - | - | H₂O _{(12% Ethanol)} | Aspergillus brasiliensis | 8,0 x 10² | 1,2 x 10⁴ | +1,18 |
| I-3 | 1,0% | H₂O | Aspergillus brasiliensis | 2,8 x 10⁴ | 1,4 x 10³ | -1,30 |
| I-3 | 0,5% | H₂O | Aspergillus brasiliensis | 2,8 x 10⁴ | 1,6 x 10⁴ | -0,24 |
| - | - | H₂O | Aspergillus brasiliensis | 2,8 x 10⁴ | 3,1 x 10⁵ | +1,04 |
| I-1 | 1,0% | H₂O | Malassezia furfur | 4,0 x 10² | 0 | -2,60 |
| - | - | H₂O | Malassezia furfur | 4,0 x 10² | 3,0 x 10⁴ | +1,86 |
| I-1 | 0,25% | H₂O | Candida albicans | 1,3 x 10⁶ | 4,3 x 10⁶ | +0,53 |
| Phenoxy ethanol | 0,5% | H₂O | Candida albicans | 1,3 x 10⁶ | 2,1 x 10⁵ | -0,78 |
| I-1 Phenoxy ethanol | 0,25% | H₂O | Candida albicans | 1,3 x 10⁶ | 0 | -6,10 |
| | 0,5% | | | | | |
| I-9 | 0,25% | H₂O | Candida albicans | 1,3 x 10⁶ | 1,4 x 10⁶ | +0,04 |
| Phenoxy ethanol | 0.5% | H₂O | Candida albicans | 1,3 x 10⁶ | 2,1 x 10⁵ | -0,78 |
| I-9 Phenoxy ethanol | 0,25% | H₂O | Candida albicans | 1,3 x 10⁶ | 0 | -6,10 |
| | 0,5% | | | | | |
| Phenoxy ethanol | 0,5% | H₂O | Aspergillus brasiliensis | 4,0 x 10⁴ | 3,0 x 10³ | -1,12 |
| I-1 Phenoxy ethanol | 0,25% | H₂O | Aspergillus brasiliensis | 4,0 x 10⁴ | 5,0 x 10² | -1,90 |
| | 0,5% | | | | | |
| I-9 Phenoxy ethanol | 0,5% | H₂O | Aspergillus brasiliensis | 4,0 x 10⁴ | 0 | -4,60 |
| | 0,5% | | | | | |
| Phenoxy ethanol | 0,5% | H₂O | Pseudomonas aeruginosa | 2,4 x 10⁶ | 2,3 x 10⁴ | -2,02 |
| I-1 Phenoxy ethanol | 0,5% | H₂O | Pseudomonas aeruginosa | 2,4 x 10⁶ | 0 | -6,38 |
| | 0,5% | | | | | |
| I-9 Phenoxy ethanol | 0,5% | H₂O | Pseudomonas aeruginosa | 2,4 x 10⁶ | 0 | -6,38 |
| | 0,5% | | | | | |
| I-1 | 0,25% | H₂O | Echerichia coli | 3,2 x 10⁵ | 8,0 x 10⁸ | +3,40 |
| Phenoxy ethanol | 0,5% | H₂O | Echerichia coli | 3,2 x 10⁵ | 2,2 x 10⁵ | -0,16 |
| I-1 Phenoxy ethanol | 0,25% | H₂O | Echerichia coli | 3,2 x 10⁵ | 7,3 x 10² | -2,64 |
| | 0,5% | | | | | |
| I-9 | 0,25% | H₂O | Echerichia coli | 3,2 x 10⁵ | 4,30 x 10⁸ | +3,13 |
| Phenoxy ethanol | 0,5% | H₂O | Echerichia coli | 3,2 x 10⁵ | 2,2 x 10⁵ | -0,16 |
| I-9 Phenoxy ethanol | 0,5% | H₂O | Echerichia coli | 3,2 x 10⁵ | 0 | -5,51 |
| | 0,5% | | | | | |

### Beispiel 3: Synthese von 2-[4-(2-Hydroxy-ethoxy)-cyclohexyloxy]-ethanol (I-3)

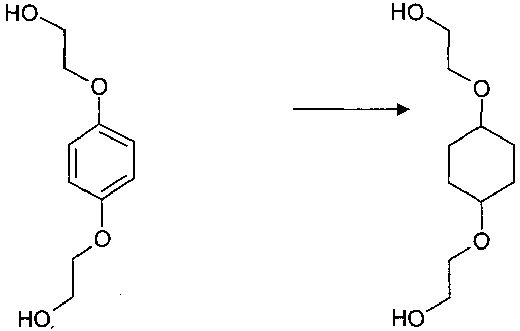

5g 2-[4-(2-Hydroxy-ethoxy)-phenoxy]-ethanol werden in 25,4mL 2-Propanol gelöst. Anschließend werden 1,2g Katalysator Pd-C-5% (ca. 50% Wasser) zugegeben. Die Hydrierung erfolgt mit Wasserstoff 3.0 bei 90-130°C und 20bar. Nach vollständiger Hydrierung wird der Katalysator durch Filtration abgetrennt. Das Filtrat wird im Vakuum vom Lösungsmittel befreit. Man erhält 5,1g Produkt im Gemisch von cis- und trans-Isomeren.

### Beispiel 4: Synthese von 2-(4-methoxy-cyclohexyloxy)-ethanol (I-5)

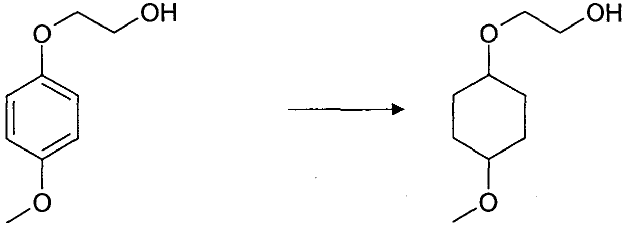

30g 2-(4-Methoxy-phenoxy)-ethanol werden in 100mL Tetrahydrofuran gelöst. Anschließend werden 3,0g Katalysator Rh-C-5% (ca. 50% Wasser) zugegeben. Die Hydrierung erfolgt mit Wasserstoff 3.0 bei 65°C und 5bar. Nach vollständiger Hydrierung wird der Katalysator durch Filtration abgetrennt. Das Filtrat wird im Vakuum vom Lösungsmittel befreit. Man erhält 25g Produkt im Gemisch von cis- und trans-Isomeren.

### Beispiel 5: Synthese von 2-tert-Butyl-4-methoxy-cyclohexanol (Verbindung I-6)

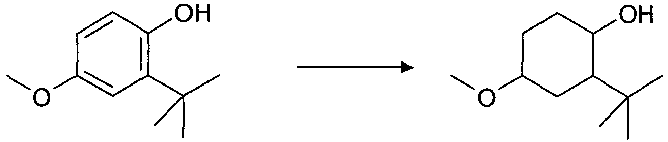

1g 2-tert-Butyl-4-methoxy-phenol werden in 50mL Ethylacetat gelöst. Anschließend wird 1g Katalysator Rh-C-5% (50% Wasser) zugegeben. Die Hydrierung erfolgt mit Wasserstoff 3.0 bei 40°C und 2bar Druck. Nach vollständiger Hydrierung wird der Katalysator durch Filtration abgetrennt und die Lösung einrotiert. Man erhält 870mg Produkt (cis- und trans-Isomeren Gemisch).

### Beispiel 6: Messung der dynamischen Viskositäten zur Abschätzung der Spreitbarkeit

Nach Messung der Dichten und kinematischen Viskositäten mit dem Ubbelohde-Viskosimeter ergeben sich folgende dynamische Viskositäten:
Kinematische Viskosität von I-1: 15,2 mm²/s
Dichte von I-1: 0,9897 g/cm³; 25°C
Dynamische Viskosität I-1 = Kinematische Viskosität I-1 x Dichte I-1 = 15,0 [mPas]

Da Spreitbarkeiten und Viskositäten kosmetischer Emollients gut korrelieren, lässt sich aus den ermittelten Viskositäten auf gute Spreitbarkeiten schließen.

### Beispiel 7: Antischuppenschampoo

| INCI | % (w/w) | % (w/w) | % (w/w) | % (w/w) | % (w/w) | % (w/w) |
|---|---|---|---|---|---|---|
| Acrylates/C10-30 Alkyl Acrylate Crosspolymer | 1,0 | 1,0 | 1,0 | 1,0 | 1,0 | 1,0 |
| Propylenglycol | 5,0 | 5,0 | 5,0 | 5,0 | 5,0 | 5,0 |
| Sodium Lauryl Sulfate (30%) | 15,0 | 15,0 | 15,0 | 15,0 | 15,0 | 15,0 |
| Sodium Laureth Sulfate (28%) | 15,0 | 15,0 | 15,0 | 15,0 | 15,0 | 15,0 |
| Cocamidopropyl betaine (35%) | 5,0 | 5,0 | 5,0 | 5,0 | 5,0 | 5,0 |
| Octopirox | 1,0 | | | | | |
| Substanz I-1 | 0,5 | 0,5 | | 1,0 | | |
| Substanz I-2 | | 0,5 | | | 0,75 | |
| Substanz I-3 | | | 0,5 | 1,0 | | |
| Substanz I-4 | | | 0,5 | | 0,75 | |
| Substanz I-5 | | 0,5 | | | 0,75 | |
| Substanz I-6 | | | 0,5 | 1,0 | | |
| Substanz I-7 | | | 0,5 | | 0,75 | |
| Substanz I-9 | 1,5 | 0,5 | 1,0 | 1,0 | 0,5 | 2,0 |
| Polyquaternium-10 | 0,30 | 0,30 | 0,30 | 0,30 | 0,30 | 0,30 |
| DMDM Hydantoin (and) Iodopropynyl Butylcarbamate | 0,3 | 0,3 | 0,3 | 0,3 | 0,3 | 0,3 |
| Polyquaternium-39 | 1,0 | 1,0 | 1,0 | 1,0 | 1,0 | 1,0 |
| Dimethicone PEG-7 Isostearate | 0,5 | 0,5 | 0,5 | 0,5 | 0,5 | 0,5 |
| Zinc Pyrithione (48%) | 2,0 | 1,0 | 1,0 | | 0,5 | 0,5 |
| Fragrance | 1,0 | 1,0 | 1,0 | 1,0 | 1,0 | 1,0 |
| Dye | 0,1 | 0,1 | 0,1 | 0,1 | 0,1 | 0,1 |
| Aqua | Ad 100 | Ad 100 | Ad 100 | Ad 100 | Ad 100 | Ad 100 |
| Sodium Hydroxide | q.s. to pH 6 | q.s. to pH 6 | q.s. to pH 6 | q.s. to pH 6 | q.s. to pH 6 | q.s. to pH 6 |

### Beispiel 8: Antiacne Gesichtsreinigungsgel

| INCI | % (w/w) | % (w/w) | % (w/w) | % (w/w) | % (w/w) | % (w/w) |
|---|---|---|---|---|---|---|
| Propylene Glycol | 2,5 | 2,5 | 2,5 | 2,5 | 2,5 | 2,5 |
| Dehydroxanthan Gum | 1,5 | 1,5 | 1,5 | 1,5 | 1,5 | 1,5 |
| Cocamidopropyl Betaine | 10,0 | 10,0 | 10,0 | 10,0 | 10,0 | 10,0 |
| Salicylic acid | 2,0 | 0,5 | 0,5 | 1,0 | | |
| Sodium Lactate | 0,5 | 1,0 | | | | 1,0 |
| Triclosan | 0,2 | | | | 0,2 | |
| 1,3.Butylene glycol | 3,5 | 3,5 | 3,5 | 3,5 | 3,5 | 3,5 |
| Octenidin | | | 0,1 | | 0,1 | |
| 1,2-Decandiol | 0,25 | 0,25 | | | 0,25 | |
| Ethylhexylglycerin | 0,25 | | 0,25 | | 0,25 | |
| Substanz I-1 | 0,5 | 0,5 | | 1,0 | 1,0 | |
| Substanz I-2 | | 0,5 | | | 0,75 | |
| Substanz I-3 | | | 0,5 | 1,0 | | |
| Substanz I-4 | | | 0,5 | | | |
| Substanz I-5 | | 0,5 | | | 0,75 | |
| Substanz I-6 | | | 0,5 | 1,0 | | |
| Substanz I-7 | | | 0,5 | | | |
| Substanz I-9 | 1,5 | 0,5 | 1,0 | 1,0 | 0,5 | 2,0 |
| Menthyl Lactate | 0,2 | 0,2 | 0,2 | 0,2 | 0,2 | 0,2 |
| PEG-40 Hydroqenated Castor Oil | 0,5 | 0,5 | 0,5 | 0,5 | 0,5 | 0,5 |
| C8-16-decyl Glucoside | 5,0 | 5,0 | 5,0 | 5,0 | 5,0 | 5,0 |
| DMDM Hydantoin (and) Iodopropynyl Butylcarbamate | 0,5 | 0,5 | 0,5 | 0,5 | 0,5 | 0,5 |
| Lactose (and) Cellulose (and) Hydroxypropyl Methylcellulose (and) Ultrmarine Blue (CI77007) (and) Triclosan | 0,4 | 0,4 | 0,4 | 0,4 | 0,4 | 0,4 |
| Fragrance | 0,5 | 0,5 | 0,5 | 0,5 | 0,5 | 0,5 |
| Aqua | Ad 100 | Ad 100 | Ad 100 | Ad 100 | Ad 100 | Ad 100 |

### Beispiel 9: Antitranspirant Spray (Aerosol)

a) Suspensionsherstellung

| INCI | % (w/w) | % (w/w) | % (w/w) | % (w/w) | % (w/w) | % (w/w) |
|---|---|---|---|---|---|---|
| Aluminium Chlorohydrate | 35,0 | 25,0 | 20,0 | 10,0 | 25,0 | |
| Ethylhexylglycerin | 0,5 | 0,5 | 0,5 | 0,5 | 0,5 | 0,5 |
| C12-15 Alkvlbenzoate | 5,0 | 5,0 | 5,0 | 5,0 | 5,0 | 5,0 |
| Diasteardimonium Hectorite | 5,0 | 5,0 | 5,0 | 5,0 | 5,0 | 5,0 |
| Silver citrate | 0,0015 | 0,0025 | | | | |
| Silver lactate | | | 0,0025 | 0,0015 | | |
| Propylencarbonate | 1,0 | 1,0 | 1,0 | 1,0 | 1,0 | 1,0 |
| 1,2-Decandiol | | 0,25 | | 1,0 | | |
| Substanz I-1 | | | | 1,0 | 1,5 | |
| Substanz I-2 | 0,5 | 1,0 | 2,0 | 1,0 | | 3,5 |
| Substanz I-3 | 2,0 | 1,5 | 0,5 | 1,0 | | 1,0 |
| Substanz I-4 | | | | 1,0 | 1,5 | |
| Substanz I-5 | 0,5 | 1,0 | 2,0 | 1,0 | | 3,5 |
| Substanz I-6 | 2,0 | 1,5 | 0,5 | 1,0 | | 1,0 |
| Substanz I-7 | | | | 1,0 | 1,5 | |
| Substanz I-9 | 1,5 | 0,5 | 1,0 | 1,0 | 0,5 | 2,0 |
| Fraqrance | 4,0 | 4,0 | 4,0 | 4,0 | 4,0 | 4,0 |
| Cyclopentasiloxane | Ad 100 | Ad 100 | Ad 100 | Ad 100 | Ad 100 | Ad 100 |

b) Treibgasgemisch aus Propan/Isobutan/Butan (1/1/1)
c) Die Aerosolherstellung erfolgt durch Verpackung der Suspension in eine Aerosoldose zusammen mit dem Treibgas im Verhältnis
Suspension/Treibgas = 20/80.

### Beispiel 10: Antiperspirant / Deodorant (Gel)

| Ingredients | % (w/w) | % (w/w) | % (w/w) | % (w/w) | % (w/w) | % (w/w) |
|---|---|---|---|---|---|---|
| Aluminium Zirconium Octachlorohydrex GLY | 10,0 | 15,0 | 20,0 | 10,0 | 15,0 | |
| Alcohol denat. | 5,0 | 5,0 | 5,0 | 5,0 | 5,0 | 5,0 |
| Cyclomethicone | 15,0 | 15,0 | 10,0 | 20,0 | 10,0 | 20,0 |
| Propylene Glycol | 10,0 | 5,0 | 5,0 | 15,0 | 5,0 | 15,0 |
| Dimethicone | 2,5 | 2,5 | 2,5 | 2,5 | 2,5 | |
| Trisiloxane | 5,0 | 2,5 | 2,5 | 5,0 | 2,5 | 5,0 |
| Octenidin | | | 0,1 | | | 0,1 |
| 1,2-Decandiol | 0,25 | 0,25 | | 0,25 | 0,25 | |
| Ethylhexylglycerin | 0,25 | | 0,25 | | 0,25 | |
| Substanz I-1 | | | | 1,0 | 1,5 | |
| Substanz I-2 | 0,5 | 1,0 | 2,0 | 1,0 | | |
| Substanz I-3 | 2,0 | 1,5 | 0,5 | 1,0 | | |
| Substanz I-4 | | | | 1,0 | 1,5 | |
| Substanz I-9 | 1,5 | 0,5 | 1,0 | 1,0 | 0,5 | 2,0 |
| Maltosin | 0,1 | 0,1 | 0,1 | 0,1 | 0,1 | 0,5 |
| Calcium Chloride | 0,3 | 0,3 | 0,3 | 0,3 | 0,3 | 0,3 |
| PEG/PPG-18/18 Dimethicone | 0,75 | 0,75 | 0,75 | 0,75 | 0,75 | 0,75 |
| Fragrance | qs | qs | qs | qs | qs | qs |
| Water | Ad 100 | Ad 100 | Ad 100 | Ad 100 | Ad 100 | Ad 100 |

### Beispiel 11: Antiperspirant / Deodorant (Roll-on)

| Ingredients | % (w/w) | % (w/w) | % (w/w) | % (w/w) | % (w/w) | % (w/w) |
|---|---|---|---|---|---|---|
| Aluminium Chlorohydrate | 25,0 | 25,0 | 25,0 | 25,0 | 25,0 | 25,0 |
| Isoceteth-20 | 1,5 | 1,5 | 1,5 | 1,5 | 1,5 | 1,5 |
| Mineral Oil | 3,0 | 3,0 | 3,0 | 3,0 | 3,0 | 3,0 |
| Butylene Glycol | 2,0 | 2,0 | 2,0 | 2,0 | 2,0 | 2,0 |
| Gylceryl Isostearate | 1,5 | 1,5 | 1,5 | 1,5 | 1,5 | 1,5 |
| Laureth-7 Citrate | 1,0 | 1,0 | 1,0 | 1,0 | 1,0 | 1,0 |
| Palmitamidopropyl Trimonium Chloride | 0,3 | 0,3 | 0,3 | 0,3 | 0,3 | 0,3 |
| Propylene Glycol | 0,5 | 0,5 | 0,5 | 0,5 | 0,5 | 0,5 |
| PEG-150 Distearate | 0,3 | 0,3 | 0,3 | 0,3 | 0,3 | 0,3 |
| Substanz I-1 | | | | 1,0 | 1,5 | |
| Substanz I-2 | 0,5 | 1,0 | 2,0 | 1,0 | | 3,5 |
| Substanz I-3 | 2,0 | 1,5 | 0,5 | 1,0 | | 1,0 |
| Substanz I-4 | | | | 1,0 | 1,5 | |
| Calcium Chloride | 0,3 | 0,3 | 0,3 | 0,3 | 0,3 | 0,3 |
| PEG/PPG-18/18 Dimethicone | 0,75 | 0,75 | 0,75 | 0,75 | 0,75 | 0,75 |
| Fragrance | qs | qs | qs | qs | qs | qs |
| Water | Ad 100 | Ad 100 | Ad 100 | Ad 100 | Ad 100 | Ad 100 |

### Beispiel 12: Anti-Aknemittel

Nachfolgende Zusammensetzungsvarianten enthalten erfindungsgemäße Stoffe in Einsatzkonzentration von jeweils zwischen 0,1 und 10 Gewichtsprozenten. Die Inhaltsstoffe werden nach INCI angegeben.
Variante 1)
   water, TEA-cocoate, cocamide DEA, potassium cocoate, TEAlaurate/myristate, butylene glycol, glycerin, glycol distearate, PEG-55 stearate, sodium cocoamphoacetate, chocolate vine (akebia quinata) root extract, scutellaria baicalensis root extract, chinese blackberry extract, angelica acutiloba root extract, coix lacryma-jobi (job's tears) seed extract, sodium polyaspartate, melothria heterophylla extract, disodium EDTA, trisodium EDTA, ethanol, xanthan gum, citric acid, sodium methyl cocoyl taurate, squalane, Hydroxypropyl Methylcellulose, polyquaternium-10, lauryl betaine, sodium bisulfite, sodium chloride, phenoxyethanol, methylparaben, fragrance
Variante 2)
   Aqua, Cyclohexasiloxane, Isononyl Isononanoate, Propylene Glycol, Isohexadecane, Niacinamide, PEG_100 stearate, Glyceryl Stearate, Cetyl Alcohol, Kaolin, Salicylic Acid, proctone olamine, Acrylates Copolymer, PEG-4, PEG-4 Dilaurate, PEG-4 Laurate, Sodium Carbomer, Capryloyl Glycine, Capryloyl Salicylic Acid, Xanthan Gum, Isobutane, Sodium Sulfate, Iodopropynyl Butylcarbamate, Chlorhexidine Digluconate, Parfum (Perfuming)
Variante 3)
   Aqua, Nylon-66, Glycerin, Cyclohexasiloxane, aluminum starch octenylsucinate, PEG-2 Stearate, Prunus Armeniaca Fruit, dydrogenated polyisobutene, Cetearyl Alcohol, Triethanolamine, Salicylic Acid, Silica, Kaolin, PEG-100 Stearate, C13-14 Isoparaffin, Stearyl Alcohol, Hamamelis Virginiana Extract, hamaelis virginiana, glyceril stearate, Sarcosine, c'glycine soja, Methylparaben, Arginine PCA, Phenoxyethanol, Cinnamomum Zeylanicum Leaf Extract, Tocopherol, Alcohol, Disodium EDTA, Capryloyl Glycine, Laureth-7, oleth-1, Acrylates/stearyl Acrylate/dimethicone Methacrylate Copolymer, Polyacrylamide, Butylene Glycol, Cl 42090, Parfum (Perfuming)
Variante 4)
   Aqua, Glycerin, Cyclohexasiloxane, Hydrogenated Polyisobutene (Hydrogenated), Niacinamide, Isopropyl Lauroyl Sarcosinate, Ammonium Polyacryloyldimethyl Taurate, Silica, Methyl Methacrylate Crosspolymer, Sodium Hydroxide, Salicylic Acid, Nylon-12, Zinc PCA, Linoleic Acid, Pentaerythrityl Tetra-di-t-butyl Hydroxyhydrocinnamate, Capryloyl Glycine, Capryloyl Salicylic Acid, Caprylyl Glycol, Piroctone Olamine, Myristyl Myristate, Potassium Cetyl Phosphate, Glyceryl Stearate SE, Parfum (Perfuming), F.I.L B36611/1
Variante 5)
   Salicylic Acid, (Aqua), D1-C 12-13 alkyl malate, Cyclohexasiloxane, Propylene Glycol, Aluminum Starch Octenylsuccinate, PEG-100 Stearate, Glyceryl Stearate, Cetyl Alcohol, PEG-4 Dilaurate, PEG-4 Laurate, Zinc PCA, Sodium Hydroxide, Capryloyl Salicylic Acid, Xanthan Gum, acrylates C10-30 alky acrylate crosspolymer, lodopropynyl Butylcarbamate, PEG-4, Parfum (Perfuming)
Variante 6)
   Resorcinol, Tocopherol, acetate, Stearyl Glycyrrhetinate, Pyridoxine HCl, Cinchona Succirubra Bark Extract (Extract), Glycerin (Concentrate), Butylene Glycol, Carbomer, Hydroxypropyl Methylcellulose, Propylene Glycol Alginate, polyoxyethylene hydrogenated castor oil, Alcohol, Triethanolamine, Aqua (Purified), Disodium EDTA, Methylparaben, Propylparaben, BHT, Parfum (Perfuming)
Variante 7)
   biosulfur fluid, Dipotassium Glycyrrhizate, Aqua, Butylene Glycol, hydrolysed rice leaf extract, Phellodendron Amurense Bark Extract (Extract), Hydroxyproline, Salvia Officinalis Leaf Extract (Extract), Camellia Sinensis Leaf (Extract), Rosmarinus Officinalis Extract (Extract), Glycerin (Cosmetic Grade), Pentylene Glycol, Carbomer, Xanthan Gum, Camphor, Menthol, Potassium Hydroxide Alcohol Denat., Aqua, Acrylates/C10-30 Alkyl Acrylate Crosspolymer, Hamamelis Virginiana Extract, Laminaria Saccharina Extract (Extract), Salicylic Acid, Sucrose, Caffeine, Butylene Glycol, Benzalkonium Chloride, ILN 32308
Variante 8)
   Aqua, Alcohol Denat., Glycerin, Acrylates/C10-30 Alkyl Acrylate Crosspolymer, Triethanolamine, Salicylic Acid, Dipotassium Glycyrrhizate, Copper Gluconate, Zinc Gluconate, Potassium Alum, Vitreoscilla Ferment (Extract), Pentylene Glycol, Sodium Citrate, Parfum (Perfuming), Limonene, Benzyl Benzoate, Linalool, Alpha-isomethyl lonone, F.I.L. B28365/2
Variante 9)
   Alcohol, Aqua, Polyacrylamide, C13-14 Isoparaffin, Laureth-7, Hamamelis Virginiana Water, Glycerin, Salicylic Acid, Cyclopentasiloxane, C12-15 Alkyl Lactate, Phenoxyethanol, Cetyl Lactate, dipropylene glycol isoceteth-20 acetate, Cocamidopropyl PG-dimonium Chloride Phosphate, Polysorbate 20, Phenethyl Dimethicone, Dehydroxanthan Gum, Parfum (Perfuming), Benzalkonium Chloride, Propylene Glycol, Ammonium Hydroxide, T-butyl Alcohol, Tetrasodium EDTA, Capryloyl Glycine, Sarcosine, Cinnamomum Zeylanicum Bark Extract (Extract), Portulaca Oleracea Extract (Extract), Cedrus Atlantica Bark Extract (Extract), Denatonium Benzoate
Variante 10)
   Alcohol, Aqua, Propylene Glycol, PEG-6, Salicylic Acid, PPG-33 Butyl Ether, Potassium Hydroxide, Benzophenone-1, Parfum (Perfuming), BHT, Isopropyl Myristate, Disodium EDTA, Lysine Carboxymethyl Cysteinate, Isododecane, Aloe Barbadensis Leaf Juice, Sodium C8-16 Isoalkylsuccinyl Lactoglobulin Sulfonate, Polystyrene/hydrogenated Polyisopentene Copolymer (Hydrogenated), Chamomilla Recutita Flower Extract (Extract), CI 42090, CI 17200
Variante 11)
   Aqua, Polyethylene, Hydrogenated Vegetable Oil (Hydrogenated, Vegetable Based), Hydrogenated Polydecene (Hydrogenated), Stearyl Alcohol, Glycerin, Glyceryl Stearate, Alcohol, PEG-40 Stearate, C12-15 Pareth-12, Cetyl Alcohol, Hamamelis Virginiana Extract (Extract), Menthol, Parfum (Perfuming), Benzethonium Chloride, Sorbic Acid, BHT, CI 15985, Eucalyptus Globulus Leaf Extract (Extract), CI 47005, CI 42090, Disodium EDTA
Variante 12)
   Aqua, Alcohol, PPG-5-ceteth-20, Glycerin, Salicylic Acid, Parfum (Perfuming), Methyl Lactate, Denatonium Benzoate
Variante 13)
   Aqua, Alcohol Denat., Dipropylene Glycol, Glycerin, Salicylic Acid, Laminaria Saccharina Extract
Variante 14)
   Polyoxyethylene ethers, Lauramine Oxide, Polyethylene (Granules), Propylene Glycol, benzalconium chlorate, Alcohol, Potassium Sorbate, Silica, yellow CI 47005, Parfum (Perfuming), Aqua (Purified)
Variante 15)
   Aqua, sodium sulfonate C14-16 olefin, Cocamidopropyl Betaine, salicilic acid, Glycerin, Decyl Glucoside, alkyl C12-13 potassium phosphate, Acrylates Copolymer, glycol distearate/ laureth-4/ cocamidopropyl betaine, Parfum (Perfuming), Citric Acid, portulaca oleracea (little hogweed) extract, Cedrus Atlantica Bark Extract (Extract), caprylyl glycine / sarcosine / cinnamomum zeylanicum (ceylon cinnamon) bark extract, Sodium Hydroxide
Variante 16)
   Aqua, Kaolin, Glycerin, Butylene Glycol, Zea Mays Starch, CI 77891, Decyl Glucoside, Polyethylene, Sodium Laureth Sulfate, Chondrus Crispus, PEG-7 Glyceryl Cocoate, Jojoba Esters, Phenoxyethanol, Salicylic Acid, Tetrasodium EDTA, Xanthan Gum, Triethanolamine, Methylparaben, Zinc Gluconate, Menthol, Butylphenyl Methylpropional, CI 77007, Propylene Glycol, Limonene, Linalool, Eucalyptus Globulus Leaf Extract (Extract), Parfum (Perfuming), F.I.L. B32440
Variante 17)
   Aqua, Sodium C14-16 Olefin Sulfonate, Glycerin, Cocamidopropyl Betaine, Salicylic Acid, Sodium Methyl Cocoyl Taurate, Acrylates Copolymer, Sodium Lauroamphoacetate, Parfum (Perfuming), Citric Acid, Glycol Distearate, Sodium Chloride, Menthyl Lactate, Cocamidopropyl PG-dimonium Chloride Phosphate
Variante 18)
   Aqua, Aqua, Decyl Glucoside, Polysorbate 20, Ceteareth-60 Myristyl Glycol, Disodium Cocoamphodiacetate, Glycerin, Sodium Lauroyl Sarcosinate, Methyl Gluceth-20, Benzoic Acid, Cetrimonium Bromide, curcurbita pepo seed oil, Disodium EDTA, Parfum (Perfuming), CI 61570, Lactic Acid, Phenoxyethanol, CI 19140, Zinc Gluconate
Variante 19)
   Aqua, Cyclomethicone, Propylene Glycol, Glycerin, Polyacrylamide, Polymethyl Methacrylate, Aqua, C13-14 Isoparaffin, Zinc Gluconate, Butylparaben, Cetrimonium Bromide, cucurbita pepo (pumpkin) seed oil (cucurbita pepo), Dimethiconol, Disodium EDTA, Parfum (Perfuming), Laureth-7, Pyridoxine HCl, Salicylic Acid, Triethanolamine
Variante 20)
   Aqua, Cetyl Alcohol, Cyclomethicone, Polysorbate 60, Glycolic Acid, Aqua, SD Alcohol 39-C, Polymethyl Methacrylate, Sodium Hydroxide, Cetearyl Alcohol, Arginine HCl, BHT, Ceteareth-33, Dimethiconol, Parfum (Perfuming), Potassium Sorbate, CI 17200, retinal undecyl rhamnoside
Variante 21)
   Aqua, Cyclohexasiloxane, Isononyl Isononanoate, Propylene Glycol, Isohexadecane, Niacinamide, PEG_100 stearate, Glyceryl Stearate, Cetyl Alcohol, Kaolin, Salicylic Acid, proctone olamine, Acrylates Copolymer, PEG-4, PEG-4 Dilaurate, PEG-4 Laurate, Sodium Carbomer, Capryloyl Glycine, Capryloyl Salicylic Acid, Xanthan Gum, Isobutane, Sodium Sulfate, lodopropynyl Butylcarbamate, Chlorhexidine Digluconate, Parfum (Perfuming)
Variante 22)
   Aqua, Dipropylene Glycol, SD Alcohol 39-C, Zinc Gluconate, cucurbita pepo pump-kin seed oil (cucurbita pepo), Parfum (Perfuming), PEG-40 Hydrogenated Castor Oil (Hydrogenated), PPG-26-buteth-26, Salicylic Acid, Silica, Stearalkonium Hectorite, Triethanolamine, Aqua
Variante 23)
   Aqua, Glycerin, Kaolin, Bentonite, Sodium Methyl Cocoyl Taurate, CI 77891, Trideceth-9, Salicylic Acid, C12-15 Alkyl Lactate, Sodium Chloride, Menthol, PEG-5 Ethylhexanoate, Xanthan Gum, Cetyl Lactate, Cocamidopropyl PG-dimonium Chloride Phosphate, Coconut Acid (Coconut Derived), Citric Acid, Sodium Citrate, Lactic Acid, Disodium EDTA, Methylparaben, Chlorphenesin, Propylparaben, Benzalkonium Chloride, Ethylparaben, Parfum (Perfuming)
Variante 24)
   Aqua, Cocamidopropyl Betaine, Sodium Myreth Sulfate, Acrylates Copolymer, Lactic Acid, Maris Limus Extract, ostera, Lauryl Glucoside, PEG-40 Hydrogenated Castor Oil (Hydrogenated), PEG-200 Hydrogenated Glyceryl Palmate (Hydrogenated), Polyethylene, Mannitol, Trisodium EDTA, Polyquaternium-10, Benzophenone-4, Microcrystalline Cellulose, Propylene Glycol, Phenoxyethanol, Methylparaben, Propylparaben, Alpha-isomethyl Ionone, Citronellol, Hexyl Cinnamal, Benzyl Salicylate, Butylphenyl Methylpropional, Parfum (Perfuming), CI 42090, CI 74160
Variante 25)
   Aqua, Sodium Laureth Sulfate, PEG-8, Coco-betaine, Hexylene Glycol, Sodium Chloride, PEG-120 Methyl Glucose Dioleate, Zinc PCA, Sodium Hydroxide, Citric Acid, Sodium Benzoate, Phenoxyethanol, Caprylyl Glycol, Parfum (Perfuming), F.I.L. B32026/1
Variante 26)
   Aqua, Kaolin, Glycerin, Butylene Glycol, Zea Mays Starch, CI 77891, Decyl Glucoside, Polyethylene, Sodium Laureth Sulfate, Chondrus Crispus, PEG-7 Glyceryl Cocoate, Salicylic Acid, Eucalyptus Globulus Leaf Extract (Extract), Menthol, Zinc Gluconate, Jojoba Esters, Propylene Glycol, Triethanolamine, Xanthan Gum, Tetrasodium EDTA, Methylparaben, Phenoxyethanol, CI 7707
Variante 27)
   Aqua, Alcohol Denat., Sorbitol, Cocamidopropyl Betaine, Parfum (Perfuming), Allantoin, Sodium Chloride, Propylene Glycol, Laminaria Digitata Extract (Extract)
Variante 28)
   Aqua, Sodium Myreth Sulfate, Disodium Laureth Sulfosuccinate, Cocamidopropyl Betaine, PEG-150 Distearate, Coco-glucoside, Glyceryl Oleate, Hexylene Glycol, Sorbitol, Cymbopogon Schoenanthus Extract (Extract), 1,10-decanediol, 10-hydroxydecanoicAcid, SebacicAcid, Parfum (Perfuming), PEG-7 Glyceryl Cocoate, Tocopherol, Hydrogenated Palm Glycerides Citrate (Hydrogenated), Propylene Glycol, Butylene Glycol, Citric Acid, Sodium Chloride, Sodium Salicylate, Sodium Benzoate
Variante 29)
   Aqua, PPG-15 Stearyl Ether, Glycerin, Stearyl Alcohol, Cetyl Betaine, Salicylic Acid, Distearyldimonium Chloride, Polyethylene, Sodium Lauryl Sulfate, Cetyl Alcohol, Alcohol, Steareth-21, Sodium Chloride, Sodium Hydroxide, Synthetic Wax (Artificial), Behenyl Alcohol, PPG-30, Steareth-2, Parfum (Perfuming), Dipropylene Glycol, Mica, Benzyl Salicylate, Limonene, Disodium EDTA, BHT, CI 77891, CI 77510
Variante 30)
   Aqua, Alcohol Denat., Glucosamine, Sorbitol, Sea Whip Extract (Extract), Cl 77120, 10-hydroxydecanoic Acid, silica disodium EDTA, benzalkonium chloride (ILN32341) nylon-12, Salicylic Acid, Butylene Glycol, Hamamelis Virginiana Extract, Laminaria Saccharina Extract (Extract), Caffeine, Sucrose, Glycerin, acetyl
Variante 31)
   Aqua, Glycerin, Butylene Glycol, Sodium Methyl Cocoyl Taurate, Sucrose, Salicylic Acid, Disodium Phosphate, Arginine Cocoate, Laminaria Saccharina Extract (Extract), Cola Nitida Seed Extract (Extract, Seed), Caffeine, Algae Extract (Extract), Mentha Piperita Leaves, Sea Whip Extract (Extract), PEG/PPG-18/18 Dimethicone, Sodium Hyaluronate, PPG-6-decyltetradeceth-30, Lactobacillus Ferment, Stearamidopropyl Dimethylamine, Longifolene, Acetyl Glucosamine, Capryloyl Glycine, perilla aldehyde, 10-hydroxydecanoic Acid, Polyquaternium-7, beta-caryophylene, Phospholipids, Sodium Salicylate, Sodium Stearate, Disodium EDTA, Phenoxyethanol, chloroxylenol (ILN 32338)
Variante 32)
   Benzoyl Peroxide 2.5%. Inactive: Water, cyclopentasiloxane, butylene glycol, ceteareth-20, dimenthicone, sucrose, green tea leaf extract, barley extract, acetyl glucosamine, lactobacillus ferment, poria cocos sclerotium extract, laminaria saccharina extract, polymethyl methacrylate, gentian root extract, sunflower seedcake, saccharomyces lysate extract, astrocaryum murumuru butter, acrylamide/sodium acryloyldimethyltaurate copolymermyristyl alcohol, glycerin
Variante 33)
   Benzoyl Peroxide, inactive (Glycerin), Petrolatum, Paraffinum Liquidum, C12-15 Alkyl Benzoate, Aqua, Sodium Cocoyl Isethionate, Sodium C14-16 Olefin Sulfonate, Zinc Lactate, Acrylates/C10-30 Alkyl Acrylate Crosspolymer, Menthol, Parfum (Perfuming), potassium polymetaphosphate, CI 77891, Carbomer
Variante 34)
   Aqua, Sodium C14-16 Olefin Sulfonate, Cocamidopropyl Betaine, Linoleamidopropyl PG-dimonium Chloride Phosphate, Polysorbate 20, Anthemis Nobilis Flower Extract (Extract), Citrus Grandis Fruit Extract (Extract), Aloe Barbadensis Flower Extract (Extract), Chamomilla Recutita Flower Extract (Extract), C12-15 Alkyl Lactate, cocamidopropyl, PG-dimonium chloride phosphate, Polyquaternium-7, Ascorbyl Palmitate, Propylene Glycol, Sodium Benzotriazolyl Butylphenol Sulfonate, PEG-120 methyl glucose dioleate PEG-80 sorbitan laurate, Disodium EDTA, Sodium Chloride, Benzalkonium Chloride, CI 16035, CI 60725, fragrance (963-277)
Variante 35)
   Salicylic Acid, (Aqua), Cyclopentasiloxane, PEG-8, Dimethicone, Dimethicone Crosspolymer, Sodium Polyacrylate, Vinyl Dimethicone/methicone Silsesquioxane Crosspolymer, cocamidopropyl PG dimonium chloride phosphate, Cucumis Sativus Fruit Extract (Extract), Camellia Sinensis Leaf Extract (Extract), Glycerin, Panthenol, Butylene Glycol, C12-15 Alkyl Lactate, Cetyl Alcohol, Glyceryl Stearate, Cetyl Lactate, PEG 75 stearate, Ceteth-20, Steareth-20, Trideceth-6, Cyclopentasiloxane, PEG/PPG-18/18 Dimethicone, Sclerotium Gum, Laureth-23, Laureth-4, Disodium EDTA, Benzalkonium Chloride, Potassium Hydroxide, CI 61570, CI 19140, CI 42090, Parfum (Perfuming)
Variante 36)
   Benzoyl Peroxide, inactive, Aqua, Bentonite, Caprylic/Capric Triglyceride, Glycerin, Emulsifying Wax (National Formulary, Emulsifying), Polysorbate 20, Glyceryl Laurate, Cetyl Dimethicone, Magnesium Aluminum Silicate, Xanthan Gum, Sodium Citrate, Disodium EDTA, Citric Acid, Methylparaben, Propylparaben
Variante 37)
   Polyquaternium-37, Silica; Aqua, Glycerin, Polysilicone-13, PEG-12 Dimethicone, Hamamelis Virginiana Extract (Extract), CI 77891, Stearyl Glycyrrhetinate, Butylene Glycol, Methylparaben
Variante 38)
   Salicylic Acid, Aqua, Sodium Cocoyl Isethionate, Cetearyl Alcohol, Laureth-3, Glycerin, Coconut Acid (Coconut Derived), Sodium Isethionate, Sodium Hydroxide, Parfum (Perfuming), Lavandula Stoechas Extract (Extract), Helichrysum Italicum Extract (Extract), Cistus Monspeliensis Extract (Extract), Disodium EDTA, PEG-40 Hydrogenated Castor Oil (Hydrogenated), Phenoxyethanol, Methylparaben, Butylparaben, Ethylparaben, Isobutylparaben, Propylparaben
Variante 39)
   Aqua, Ethylhexyl Methoxycinnamate, Ethylhexyl Salicylate, Benzophenone-3, Butyl Methoxydibenzoylmethane, Propylene Glycol, C12-15 Alkyl Benzoate, Dimethicone, Glyceryl Stearate, Thiodipropionic Acid, Retinyl Palmitate, Triticum Vulgare Germ Extract (Extract, Germs), Saccharomyces/zinc Ferment, Glycolic Acid, Salicylic Acid, Pyridoxine HCl, Tocopherol, Palmitoyl Oligopeptide, Palmitoyl Tetrapeptide-7, Glycerin, Aluminum Starch Octenylsuccinate, Hydroxyethyl Acrylate/Sodium Acryloyldimethyl Taurate Copolymer, Silica, Isohexadecane, Acrylates Crosspolymer, PEG-100 Stearate, Caprylyl Glycol, Hydrogenated Polyisobutene (Hydrogenated), Hydroxyethylcellulose, Xanthan Gum, Choleth-24, Polysorbate 60, Ceteth-24, Isododecane, Sorbitan Isostearate, Polystyrene/hydrogenated Polyisopentene Copolymer (Hydrogenated), Butylene Glycol, Carbomer, Polysorbate 20, Steareth-2, Ammonium Hydroxide, Cetyl Alcohol, Stearic Acid, Disodium EDTA, Phenoxyethanol, Parfum (Perfuming).

### Beispiel 13: Plastisole

| | **Plastisol 1** | **Plastisol 2** | **Plastisol 3** | **Plastisol 4** | **Plastisol 5** |
|---|---|---|---|---|---|
| PVC (Vestolit B 7021, Fa. Vestolit) | 100g | 100g | 100g | 100g | 100g |
| Di-iso-nonylphthalat (Vestinol 9, Fa. Evonik) | 30g | | 30g | | 30g |
| 1,2-Diisononylcyclohexanedicarboxylic acid ester | | 30g | | 30g | |
| Di-n-pentylterephthalat | 10g | | 10g | | 10g |
| 1,4-Dipentylcyclohexane-dicarboxyilc acid ester | | 10g | | 10g | |
| **Substanz I-9** | 10g | 10g | 10g | 10g | 10g |
| Epoxidiertes Sojabohnenöl | 3g | 3g | 3g | 3g | 3g |
| Mark CZ 140 (Ca/Zn-Stab., Crompton) | 1,5g | 1,5g | 1,5g | 1,5g | 1,5g |

| | **Plastisol 6** | **Plastisol 7** | **Plastisol 8** | **Plastisol 9** | **Plastisol 10** |
|---|---|---|---|---|---|
| PVC (Vestolit B 7021, Fa. Vestolit) | 100g | 100g | 100g | 100g | 100g |
| Di-iso-nonylphthalat (Vestinol 9, Fa. Evonik) | 30g | | 30g | | 30g |
| 1,2-Diisononylcyclohexanedicarboxylic acid ester | | 30g | | 30g | |
| Di-n-pentylterephthalat | 10g | | 10g | | 10g |
| 1,4-Dipentylcyclohexanedicarboxylic acid ester | | 10g | | 10g | |
| **Substanz I-16** | 10g | 10g | 10g | 10g | 10g |
| Epoxidiertes Sojabohnenöl | 3g | 3g | 3g | 3g | 3g |
| Mark CZ 140 (Ca/Zn-Stab., Crompton) | 1,5g | 1,5g | 1,5g | 1,5g | 1,5g |

## Patentansprüche

1. Nicht-therapeutische Verwendung mindestens einer Verbindung der Formel I 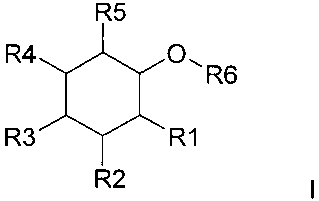
worin R1, R2, R4 und R5 unabhängig voneinander stehen für einen Rest ausgewählt aus H und t-Butyl,
worin R3 steht für einen Rest ausgewählt aus
- H, OH, OCOCH₃, O(CH₂CH₂O)ₙH wobei n = 1 bis 20, Halogen,
- geradkettige oder verzweigte Alkylgruppe mit 1 bis 20 C-Atomen, welche optional mit ein oder mehreren OH-Gruppen substituiert sein kann, und/ oder durch eine oder mehrere Gruppen ausgewählt aus -O-, -(C=O)-, -(CO)O- und Cyclohexylen unterbrochen sein kann,
- geradkettige oder verzweigte O-Alkylgruppe mit 1 bis 20 C-Atomen,
und worin R6 steht für einen Rest ausgewählt ausCH₃, CH₂CH(CH₃)OH und CH₂CH₂OH,
als antimikrobieller Wirkstoff.

2. Verwendung nach Anspruch 1 in kosmetischen Formulierungen, Lebensmitteln,Haushaltsprodukten, Kunststoffen, Papier und/ oder Farben, insbesondere in Zahn- oder Mundpflegeprodukten.

3. Verbindung der Formel I 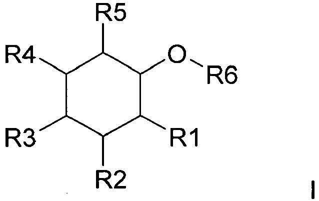
worin R1, R2, R4 und R5 unabhängig voneinander stehen für einen Rest ausgewählt aus H und t-Butyl,
worin R3 steht für einen Rest ausgewählt aus
- H, OH, OCOCH₃, O(CH₂CH₂O)ₙH wobei n = 1 bis 20, Halogen,
- geradkettige oder verzweigte Alkylgruppe mit 1 bis 20 C-Atomen, welche optional mit ein oder mehreren OH-Gruppen substituiert sein kann, und/ oder durch eine oder mehrere Gruppen ausgewählt aus -O-, -(C=O)-, -(CO)O- und Cyclohexylen unterbrochen sein kann,
- geradkettige oder verzweigte O-Alkylgruppe mit 1 bis 20 C-Atomen,
und worin R6 steht für einen Rest ausgewählt ausCH₃, CH₂CH(CH₃)OH und CH₂CH₂OH,
zur Verwendung als antimikrobieller Wirkstoff in pharmazeutischen Formulierungen und/ oder Medizinprodukten.

4. Verwendung nach Anspruch 1 oder 2 zur Konservierungsverbesserung.

5. Verwendung nach Anspruch 1 zur Konservierungsverbesserung in pharmazeutischen Formulierungen und/ oder Medizinprodukten.

6. Nicht-therapeutische Verwendung mindestens einer Verbindung der Formel I 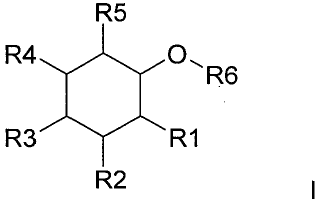
worin R1, R2, R4 und R5 unabhängig voneinander stehen für einen Rest ausgewählt aus H und t-Butyl,
worin R3steht für einen Rest ausgewählt aus
- H, OH, OCOCH₃, O(CH₂CH₂O)ₙH, wobei n = 1 bis 20, Halogen,
- geradkettige oder verzweigte Alkylgruppe mit 1 bis 20 C-Atomen, welche optional mit ein oder mehreren OH-Gruppen substituiert sein kann, und/ oder durch eine oder mehrere Gruppen ausgewählt aus -O-, -(C=O)-, -(CO)O- und Cyclohexylen unterbrochen sein kann,
- geradkettige oder verzweigte O-Alkylgruppe mit 1 bis 20 C-Atomen,
und worin R6 steht für einen Rest ausgewählt aus CH₃, CH₂CH(CH₃)OH und CH₂CH₂OH,
als Deodorant- oder Antitranspirantwirkstoff.

7. Verbindung der Formel I 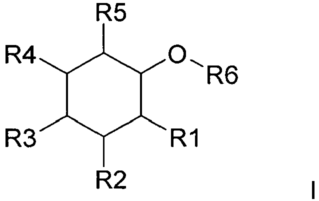
worin R1, R2, R4 und R5 unabhängig voneinander stehen für einen Rest ausgewählt aus H und t-Butyl,
worin R3steht für einen Rest ausgewählt aus
- H, OH, OCOCH₃, O(CH₂CH₂O)ₙH, wobei n = 1 bis 20, Halogen,
- geradkettige oder verzweigte Alkylgruppe mit 1 bis 20 C-Atomen, welche optional mit ein oder mehreren OH-Gruppen substituiert sein kann, und/ oder durch eine oder mehrere Gruppen ausgewählt aus -O-, -(C=O)-, -(CO)O- und Cyclohexylen unterbrochen sein kann,
- geradkettige oder verzweigte O-Alkylgruppe mit 1 bis 20 C-Atomen,
und worin R6 steht für einen Rest ausgewählt aus CH₃, CH₂CH(CH₃)OH und CH₂CH₂OH,
zur Verwendung als Anti-Akne- und/oder Antischuppenwirkstoff.

8. Verwendung nach einem oder mehreren der Ansprüche 1, 2 und 4 bis 6, oder Verbindung nach Anspruch 3 oder 7, **dadurch gekennzeichnet, dass** R3 steht für einen Rest ausgewählt aus
- H, OH, O(CH₂CH₂O)ₙH, wobei n = 1 bis 2,
- geradkettige oder verzweigte Alkylgruppe mit 1 bis 12 C-Atomen, welche optional mit ein oder mehreren OH-Gruppen substituiert sein kann, und/ oder durch eine oder mehrere Gruppen ausgewählt aus -O-, -(C=O)-, -(CO)O- und Cyclohexylen unterbrochen sein kann,
- geradkettige oder verzweigte O-Alkylgruppe mit 1 bis 12 C-Atomen.

9. Verwendung oder Verbindung nach Anspruch 8, **dadurch gekennzeichnet, dass** R3 steht für einen Rest ausgewählt aus H, OH, OCH₂CH₂OH, OCH₃ und CH₂OH.

10. Verwendung nach einem oder mehreren der Ansprüche 1, 2, 4 bis 6 und 8 oder Verbindung nach einem oder mehreren der Ansprüche 3, 7 und 8, **dadurch gekennzeichnet, dass** die Verbindung der Formel I für eine Verbindung ausgewählt aus den Verbindungen der Formeln I-1 bis I-9 steht
| | | | |
|---|---|---|---|
| I-1 | 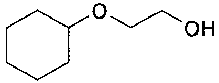 | I-2 | 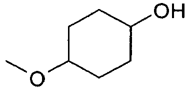 |
| I-3 | 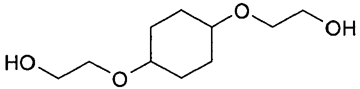 | I-4 | 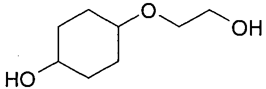 |
| I-5 | 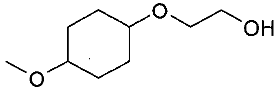 | I-6 | 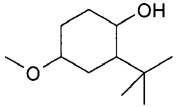 |
| I-7 | 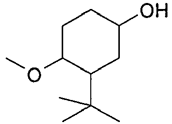 | I-8 | 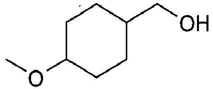 |
| I-9 | 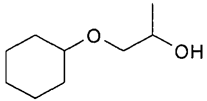 | | |

11. Zubereitung enthaltend mindestens eine Verbindung der Formel I 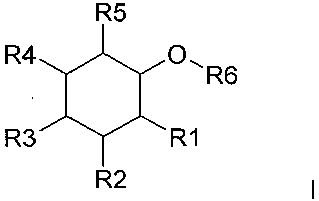
worin R1, R2, R4 und R5 unabhängig voneinander stehen für einen Rest ausgewählt aus H und t-Butyl
worin R3steht für einen Rest ausgewählt aus
- H, OH, OCOCH₃, O(CH₂CH₂O)ₙH, wobei n = 1 bis 20, Halogen,
- geradkettige oder verzweigte Alkylgruppe mit 1 bis 20 C-Atomen, welche optional mit ein oder mehreren OH-Gruppen substituiert sein kann, und/ oder durch eine oder mehrere Gruppen ausgewählt aus -O-, -(C=O)-, -(CO)O- und Cyclohexylen unterbrochen sein kann,
- geradkettige oder verzweigte O-Alkylgruppe mit 1 bis 20 C-Atomen,
und worin R6 steht für einen Rest ausgewählt aus CH₃, CH₂CH(CH₃)OH und CH₂CH₂OH,
und mindestens einen geeigneten Träger, **dadurch gekennzeichnet, dass** neben der mindestens einen Verbindung der Formel I mindestens ein weiterer Konservierungsstoff und/oder antimikrobieller Wirkstoff enthalten ist.

12. Zubereitung nach Anspruch 11, **dadurch gekennzeichnet, dass** die Zubereitung ein Deodorant, ein Antitranspirant, ein Anti-Schuppen- oder Anti-Akne-Mittel oder eine antibakterielle Zubereitung, insbesondere zur Zahn- oder Mundpflege, ist.

13. Zubereitung nach Anspruch 11 oder 12, **dadurch gekennzeichnet, dass** weiterhin mindestens ein Antioxidans enthalten ist.

14. Zubereitung enthaltend mindestens eine Verbindung der Formel I 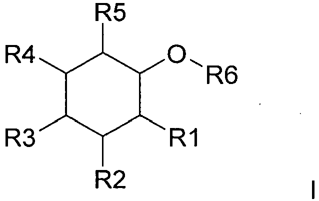
worin R1, R2, R4 und R5 unabhängig voneinander stehen für einen Rest ausgewählt aus H und t-Butyl,
worin R3steht für einen Rest ausgewählt aus
- H, OH, OCOCH₃, O(CH₂CH₂O)ₙH, wobei n = 1 bis 20, Halogen,
- geradkettige oder verzweigte Alkylgruppe mit 1 bis 20 C-Atomen, welche optional mit ein oder mehreren OH-Gruppen substituiert sein kann, und/ oder durch eine oder mehrere Gruppen ausgewählt aus -O-, -(C=O)-, -(CO)O- und Cyclohexylen unterbrochen sein kann,
- geradkettige oder verzweigte O-Alkylgruppe mit 1 bis 20 C-Atomen,
und worin R6 steht für einen Rest ausgewählt aus CH₃, CH₂CH(CH₃)OH und CH₂CH₂OH,
und mindestens ein Antioxidans.

15. Verfahren zur Herstellung einer Zubereitung nach einem oder mehreren der Ansprüche 11 bis 14, **dadurch gekennzeichnet, dass** die Verbindung der Formel I mit einem geeigneten Träger vermischt wird.

16. Verbindung der Formel I-4, I-5, I-6, I-11 bis I-16 oder I-17 bis I-20 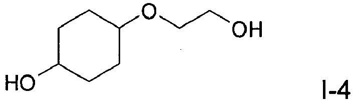 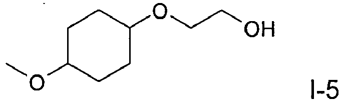 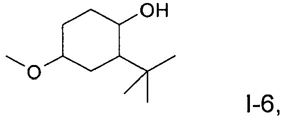
| | | | |
|---|---|---|---|
| I-11 | 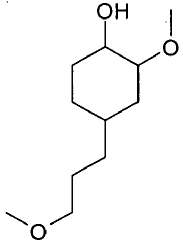 | I-12 | 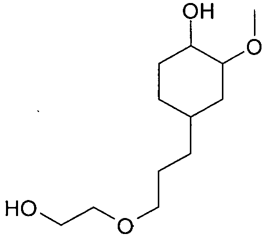 |
| I-13 | 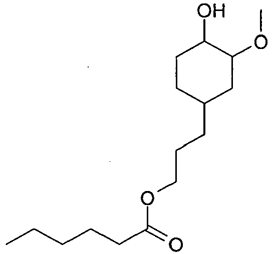 | I-14 | 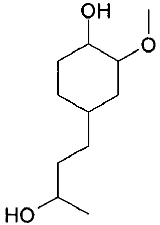 |
| I-15 | 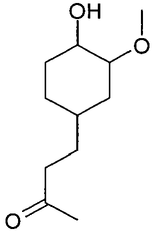 | I-16 | 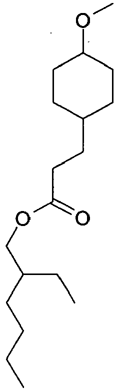 |
| I-17 | 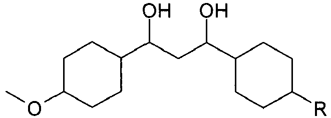 | I-18 | 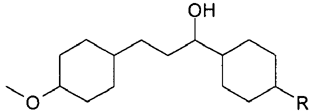 |
| I-19 | 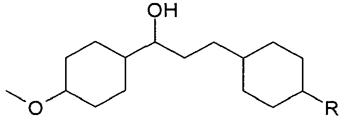 | I-20 | 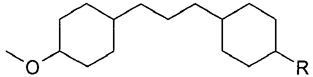 |
wobei R in den Formeln I-17 bis I-20 für H, Methoxy, t-Butyl oder Isopropyl steht und wobei I-20 mit R=H ausgeschlossen ist.

## Claims

1. Non-therapeutic use of at least one compound of the formula I 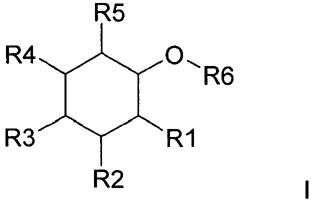
in which R1, R2, R4 and R5 stand, independently of one another, for a radical selected from H and t-butyl,
in which R3 stands for a radical selected from
- H, OH, OCOCH₃, O(CH₂CH₂O)ₙH, where n = 1 to 20, halogen,
- straight-chain or branched alkyl group having 1 to 20 C atoms, which may optionally be substituted by one or more OH groups and/or may be interrupted by one or more groups selected from -O-, -(C=O)-, -(CO)O- and cyclohexylene,
- straight-chain or branched O-alkyl group having 1 to 20 C atoms,
and in which R6 stands for a radical selected from CH₃, CH₂CH(CH₃)OH and CH₂CH₂OH,
as antimicrobial active compound.

2. Use according to Claim 1 in cosmetic formulations, foods, household products, plastics, paper and/or paints, in particular in dental or oral care products.

3. Compound of the formula I 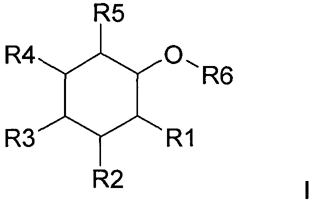
in which R1, R2, R4 and R5 stand, independently of one another, for a radical selected from H and t-butyl,
in which R3 stands for a radical selected from
- H, OH, OCOCH₃, O(CH₂CH₂O)ₙH, where n = 1 to 20, halogen,
- straight-chain or branched alkyl group having 1 to 20 C atoms, which may optionally be substituted by one or more OH groups and/or may be interrupted by one or more groups selected from -O-, -(C=O)-, -(CO)O- and cyclohexylene,
- straight-chain or branched O-alkyl group having 1 to 20 C atoms,
and in which R6 stands for a radical selected from CH₃, CH₂CH(CH₃)OH and CH₂CH₂OH,
for use as antimicrobial active compound in pharmaceutical formulations and/or medicinal products.

4. Use according to Claim 1 or 2 for improving preservation.

5. Use according to Claim 1 for improving preservation in pharmaceutical formulations and/or medicinal products.

6. Non-therapeutic use of at least one compound of the formula I 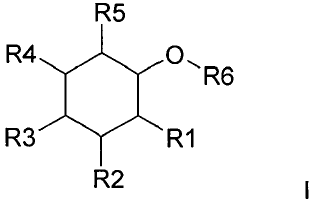
in which R1, R2, R4 and R5 stand, independently of one another, for a radical selected from H and t-butyl,
in which R3 stands for a radical selected from
- H, OH, OCOCH₃, O(CH₂CH₂O)ₙH, where n = 1 to 20, halogen,
- straight-chain or branched alkyl group having 1 to 20 C atoms, which may optionally be substituted by one or more OH groups and/or may be interrupted by one or more groups selected from -O-, -(C=O)-, -(CO)O- and cyclohexylene,
- straight-chain or branched O-alkyl group having 1 to 20 C atoms,
and in which R6 stands for a radical selected from CH₃, CH₂CH(CH₃)OH and CH₂CH₂OH,
as deodorant or antiperspirant active compound.

7. Compound of the formula I 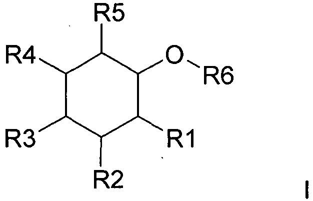
in which R1, R2, R4 and R5 stand, independently of one another, for a radical selected from H and t-butyl,
in which R3 stands for a radical selected from
- H, OH, OCOCH₃, O(CH₂CH₂O)ₙH, where n = 1 to 20, halogen,
- straight-chain or branched alkyl group having 1 to 20 C atoms, which may optionally be substituted by one or more OH groups and/or may be interrupted by one or more groups selected from -O-, -(C=O)-, -(CO)O- and cyclohexylene,
- straight-chain or branched O-alkyl group having 1 to 20 C atoms,
and in which R6 stands for a radical selected from CH₃, CH₂CH(CH₃)OH and CH₂CH₂OH,
for use as anti-acne and/or antidandruff active compound.

8. Use according to one or more of Claims 1, 2 and 4 to 6, or compound according to Claim 3 or 7, **characterised in that** R3 stands for a radical selected from
- H, OH, O(CH₂CH₂O)ₙH, where n = 1 to 2,
- straight-chain or branched alkyl group having 1 to 12 C atoms, which may optionally be substituted by one or more OH groups and/or may be interrupted by one or more groups selected from -O-, -(C=O)-, -(CO)O- and cyclohexylene,
- straight-chain or branched O-alkyl group having 1 to 12 C atoms.

9. Use or compound according to Claim 8, **characterised in that** R3 stands for a radical selected from H, OH, OCH₂CH₂OH, OCH₃ and CH₂OH.

10. Use according to one or more of Claims 1, 2, 4 to 6 and 8 or compound according to one or more of Claims 3, 7 and 8, **characterised in that** the compound of the formula I stands for a compound selected from the compounds of the formulae I-1 to I-9
| | | | |
|---|---|---|---|
| I-1 | 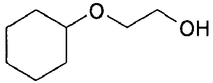 | I-2 | 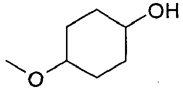 |
| I-3 | 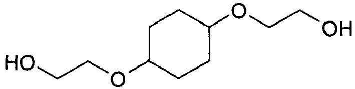 | I-4 | 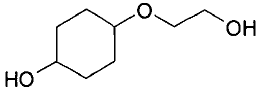 |
| I-5 | 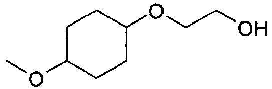 | I-6 | 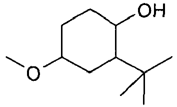 |
| I-7 | 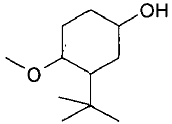 | I-8 | 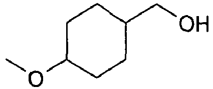 |
| I-9 | 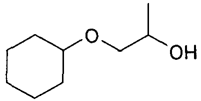 | | |

11. Preparation comprising at least one compound of the formula I 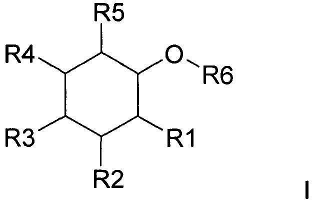
in which R1, R2, R4 and R5 stand, independently of one another, for a radical selected from H and t-butyl,
in which R3 stands for a radical selected from
- H, OH, OCOCH₃, O(CH₂CH₂O)ₙH, where n = 1 to 20, halogen,
- straight-chain or branched alkyl group having 1 to 20 C atoms, which may optionally be substituted by one or more OH groups and/or may be interrupted by one or more groups selected from -O-, -(C=O)-, -(CO)O- and cyclohexylene,
- straight-chain or branched O-alkyl group having 1 to 20 C atoms,
and in which R6 stands for a radical selected from CH₃, CH₂CH(CH₃)OH and CH₂CH₂OH,
and at least one suitable vehicle, **characterised in that**, besides the at least one compound of the formula I, at least one further preservative and/or antimicrobial active compound is present.

12. Preparation according to Claim 11, **characterised in that** the preparation is a deodorant, an antiperspirant, an antidandruff or anti-acne composition or an antibacterial preparation, in particular for dental or oral care.

13. Preparation according to Claim 11 or 12, **characterised in that** at least one antioxidant is furthermore present.

14. Preparation comprising at least one compound of the formula I 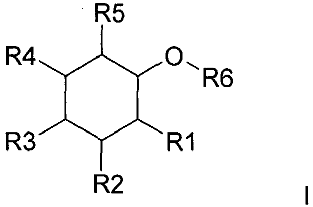
in which R1, R2, R4 and R5 stand, independently of one another, for a radical selected from H and t-butyl,
in which R3 stands for a radical selected from
- H, OH, OCOCH₃, O(CH₂CH₂O)ₙH, where n = 1 to 20, halogen,
- straight-chain or branched alkyl group having 1 to 20 C atoms, which may optionally be substituted by one or more OH groups and/or may be interrupted by one or more groups selected from -O-, -(C=O)-, -(CO)O- and cyclohexylene,
- straight-chain or branched O-alkyl group having 1 to 20 C atoms,
and in which R6 stands for a radical selected from CH₃, CH₂CH(CH₃)OH and CH₂CH₂OH,
and at least one antioxidant.

15. Process for the preparation of a preparation according to one or more of Claims 11 to 14, **characterised in that** the compound of the formula I is mixed with a suitable vehicle.

16. Compound of the formula I-4, I-5, I-6, I-11 to I-16 or I-17 to I-20 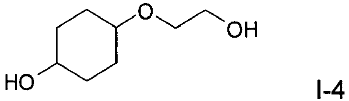 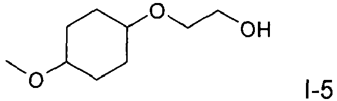 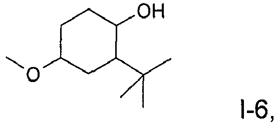
| | | | |
|---|---|---|---|
| I-11 | 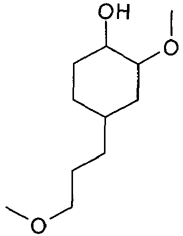 | I-12 | 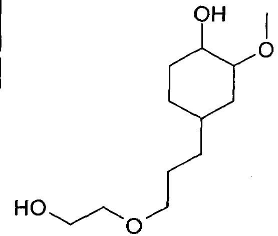 |
| I-13 | 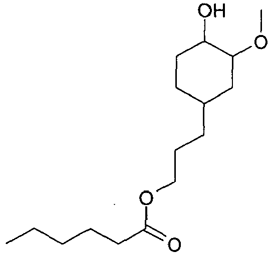 | I-14 | 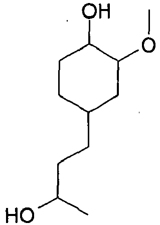 |
| I-15 | 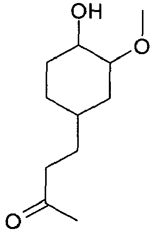 | I-16 | 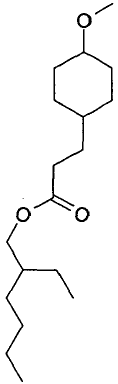 |
| I-17 | 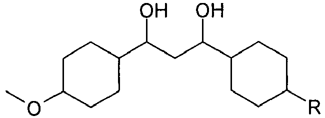 | I-18 | 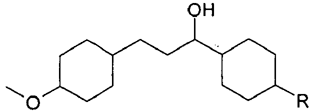 |
| I-19 | 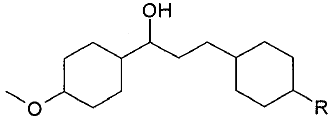 | I-20 | 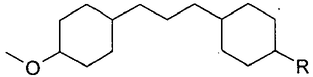 |
where R in the formulae I-17 to I-20 stands for H, methoxy, t-butyl or isopropyl and where I-20 where R=H is excluded.

## Revendications

1. Utilisation non thérapeutique d'au moins un composé de formule I 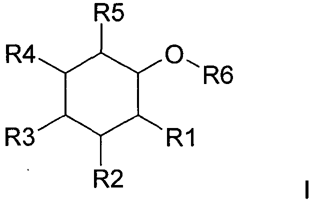
dans lequel R1, R2, R4 et R5 représentent, indépendamment les uns des autres, un radical choisi parmi H et t-butyle,
où R3 représente un radical choisi parmi
- H, OH, OCOCH₃, O(CH₂CH₂O)ₙH, où n = 1 à 20, halogène,
- un groupement alkyle à chaîne linéaire ou ramifiée ayant de 1 à 20 atomes de C, pouvant éventuellement être substitué par un ou plusieurs groupements OH et/ou pouvant être interrompu par un ou plusieurs groupements choisis parmi -O-, -(C=O)-, -(CO)O- et cyclohexylène,
- un groupement O-alkyle à chaîne linéaire ou ramifiée ayant de 1 à 20 atomes de C,
et où R6 représente un radical choisi parmi CH₃, CH₂CH(CH₃)OH et CH₂CH₂OH,
comme composé actif antimicrobien.

2. Utilisation selon la revendication 1, dans des formulations cosmétiques, des aliments, des produits d'entretien ménager, des matières plastiques, du papier et/ou des peintures, en particulier dans des produits de soin dentaire ou oral.

3. Composé de formule I 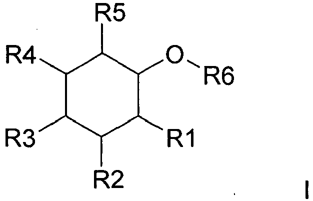
dans lequel R1, R2, R4 et R5 représentent, indépendamment les uns des autres, un radical choisi parmi H et t-butyle,
où R3 représente un radical choisi parmi
- H, OH, OCOCH₃, O(CH₂CH₂O)ₙH, où n = 1 à 20, halogène,
- un groupement alkyle à chaîne linéaire ou ramifiée ayant de 1 à 20 atomes de C, pouvant éventuellement être substitué par un ou plusieurs groupements OH et/ou pouvant être interrompu par un ou plusieurs groupements choisis parmi -O-, -(C=O)-, -(CO)O- et cyclohexylène,
- un groupement O-alkyle à chaîne linéaire ou ramifiée ayant de 1 à 20 atomes de C,
et où R6 représente un radical choisi parmi CH₃, CH₂CH(CH₃)OH et CH₂CH₂OH,
pour une utilisation comme composé actif antimicrobien dans des formulations pharmaceutiques et/ou des produits médicinaux.

4. Utilisation selon la revendication 1 ou 2, pour améliorer la conservation.

5. Utilisation selon la revendication 1, pour améliorer la conservation dans des formulations pharmaceutiques et/ou des produits médicinaux.

6. Utilisation non thérapeutique d'au moins un composé de formule I 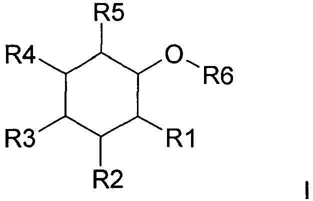
dans lequel R1, R2, R4 et R5 représentent, indépendamment les uns des autres, un radical choisi parmi H et t-butyle,
où R3 représente un radical choisi parmi
- H, OH, OCOCH₃, O(CH₂CH₂O)ₙH, où n = 1 à 20, halogène,
- un groupement alkyle à chaîne linéaire ou ramifiée ayant de 1 à 20 atomes de C, pouvant éventuellement être substitué par un ou plusieurs groupements OH et/ou pouvant être interrompu par un ou plusieurs groupements choisis parmi -O-, -(C=O)-, -(CO)O- et cyclohexylène,
- un groupement O-alkyle à chaîne linéaire ou ramifiée ayant de 1 à 20 atomes de C,
et où R6 représente un radical choisi parmi CH₃, CH₂CH(CH₃)OH et CH₂CH₂OH,
comme composé actif déodorant ou anti-transpirant.

7. Composé de formule I 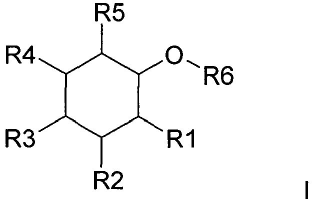
dans lequel R1, R2, R4 et R5 représentent, indépendamment les uns des autres, un radical choisi parmi H et t-butyle,
où R3 représente un radical choisi parmi
- H, OH, OCOCH₃, O(CH₂CH₂O)ₙH, où n = 1 à 20, halogène,
- un groupement alkyle à chaîne linéaire ou ramifiée ayant de 1 à 20 atomes de C, pouvant éventuellement être substitué par un ou plusieurs groupements OH et/ou pouvant être interrompu par un ou plusieurs groupements choisis parmi -O-, -(C=O)-, -(CO)O- et cyclohexylène,
- un groupement O-alkyle à chaîne linéaire ou ramifiée ayant de 1 à 20 atomes de C,
et où R6 représente un radical choisi parmi CH₃, CH₂CH(CH₃)OH et CH₂CH₂OH,
pour une utilisation comme composé actif anti-acné et/ou antipelliculaire.

8. Utilisation selon l'une ou plusieurs parmi les revendications 1, 2 et 4 à 6, ou composé selon la revendication 3 ou 7, caractérisé(e) en ce que R3 représente un radical choisi parmi
- H, OH, O(CH₂CH₂O)ₙH, où n = 1 à 2,
- un groupement alkyle à chaîne linéaire ou ramifiée ayant de 1 à 12 atomes de C, pouvant éventuellement être substitué par un ou plusieurs groupements OH et/ou pouvant être interrompu par un ou plusieurs groupements choisis parmi -O-, -(C=O)-, -(CO)O- et cyclohexylène,
- un groupement O-alkyle à chaîne linéaire ou ramifiée ayant de 1 à 12 atomes de C.

9. Utilisation ou composé selon la revendication 8, caractérisé(e) en ce que R3 représente un radical choisi parmi H, OH, OCH₂CH₂OH, OCH₃ et CH₂OH.

10. Utilisation selon l'une ou plusieurs parmi les revendications 1, 2, 4 à 6 et 8 ou composé selon l'une ou plusieurs parmi les revendications 3, 7 et 8, caractérisé(e) en ce que le composé de formule I représente un composé choisi parmi les composés de formules I-1 à I-9
| | | | |
|---|---|---|---|
| I-1 | 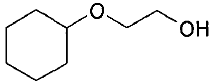 | I-2 | 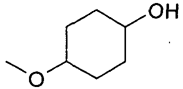 |
| I-3 | 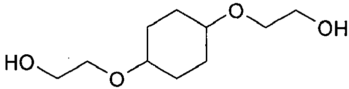 | I-4 | 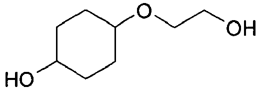 |
| I-5 | 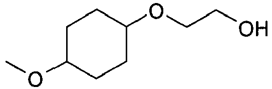 | I-6 | 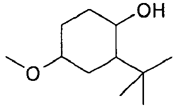 |
| I-7 | 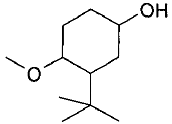 | I-8 | 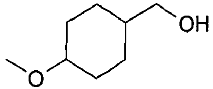 |
| I-9 | 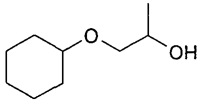 | | |

11. Préparation comprenant au moins un composé de formule I 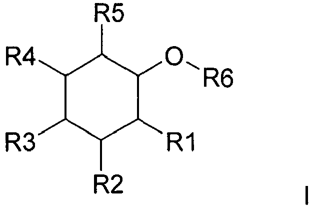
dans lequel R1, R2, R4 et R5 représentent, indépendamment les uns des autres, un radical choisi parmi H et t-butyle,
où R3 représente un radical choisi parmi
- H, OH, OCOCH₃, O(CH₂CH₂O)ₙH, où n = 1 à 20, halogène,
- un groupement alkyle à chaîne linéaire ou ramifiée ayant de 1 à 20 atomes de C, pouvant éventuellement être substitué par un ou plusieurs groupements OH et/ou pouvant être interrompu par un ou plusieurs groupements choisis parmi -O-, -(C=O)-, -(CO)O- et cyclohexylène,
- un groupement O-alkyle à chaîne linéaire ou ramifiée ayant de 1 à 20 atomes de C,
et où R6 représente un radical choisi parmi CH₃, CH₂CH(CH₃)OH et CH₂CH₂OH,
et au moins un véhicule convenable, **caractérisée en ce que**, outre le au moins un composé de formule I, au moins un autre conservateur et/ou composé actif antimicrobien est présent.

12. Préparation selon la revendication 11, **caractérisée en ce que** la préparation est un déodorant, un anti-transpirant, une composition antipelliculaire ou anti-acné ou une préparation antibactérienne, en particulier pour le soin dentaire ou oral.

13. Préparation selon la revendication 11 ou 12, **caractérisée en ce qu'**au moins un antioxydant est en outre présent.

14. Préparation comprenant au moins un composé de formule I 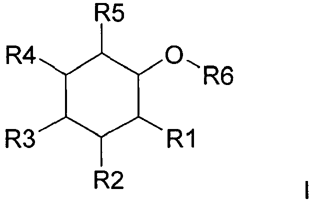
dans lequel R1, R2, R4 et R5 représentent, indépendamment les uns des autres, un radical choisi parmi H et t-butyle,
où R3 représente un radical choisi parmi
- H, OH, OCOCH₃, O(CH₂CH₂O)ₙH, où n = 1 à 20, halogène,
- un groupement alkyle à chaîne linéaire ou ramifiée ayant de 1 à 20 atomes de C, pouvant éventuellement être substitué par un ou plusieurs groupements OH et/ou pouvant être interrompu par un ou plusieurs groupements choisis parmi -O-, -(C=O)-, -(CO)O- et cyclohexylène,
- un groupement O-alkyle à chaîne linéaire ou ramifiée ayant de 1 à 20 atomes de C,
et où R6 représente un radical choisi parmi CH₃, CH₂CH(CH₃)OH et CH₂CH₂OH,
et au moins un antioxydant.

15. Procédé de préparation d'une préparation selon l'une ou plusieurs parmi les revendications 11 à 14, **caractérisé en ce que** le composé de formule I est mélangé avec un véhicule convenable.

16. Composé de formule I-4, I-5, I-6, I-11 à I-16 ou I-17 à I-20 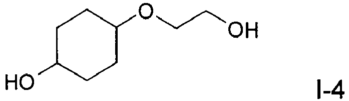 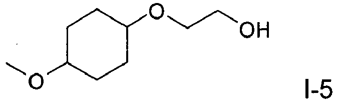 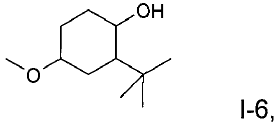
| | | | |
|---|---|---|---|
| I-11 | 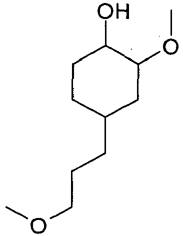 | I-12 | 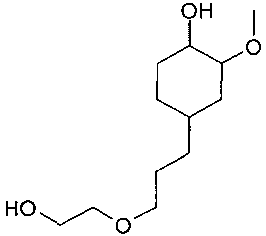 |
| I-13 | 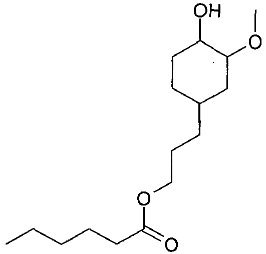 | I-14 | 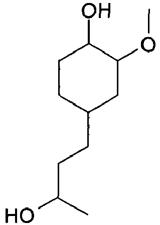 |
| I-15 | 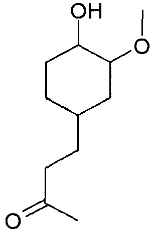 | I-16 | 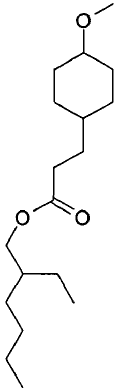 |
| I-17 | 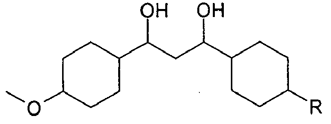 | I-18 | 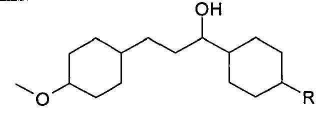 |
| I-19 | 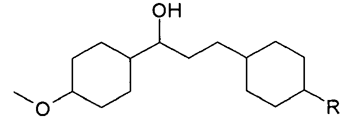 | I-20 | 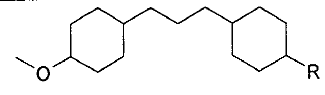 |
où R dans les formules I-17 à I-20 représente H, méthoxy, t-butyle ou isopropyle et où I-20 où R=H est exclu.
